# EUROPEAN PATENT SPECIFICATION

(11) **EP 3 122 878 B1**
(45) Date of publication and mention of the grant of the patent: **24.10.2018**
(21) Application number: 15767977.0
(22) Date of filing: 19.03.2015
(51) Int. Cl.: C12N 15/02, C12P 21/06, A61K 48/00

(54) **MRNA THERAPY FOR THE TREATMENT OF OCULAR DISEASES**
MRNA-THERAPIE ZUR BEHANDLUNG VON AUGENERKRANKUNGEN
THÉRAPIE BASÉE SUR L'ARNM POUR LE TRAITEMENT DES MALADIES OCULAIRES

(30) Priority: 24.03.2014 US 201461969483 P
(43) Date of publication of application: 01.02.2017
(73) Proprietor: Translate Bio, Inc., Lexington, MA 02421 (US)
(72) Inventor: CALIAS, Pericles, Lexington, Massachusetts 02421 (US); DEROSA, Frank, Lexington, Massachusetts 02421 (US); HEARTLEIN, Michael, Lexington, Massachusetts 02421 (US)
(74) Representative: Carpmaels & Ransford LLP
(86) International application number: PCT/US2015/021403
(87) International publication number: WO 2015/148247

(56) References cited:
- WO-A1-2012/170930
- WO-A1-2013/149140
- WO-A1-2013/151671
- WO-A2-2006/110813
- US-A1- 2006 241 071
- US-A1- 2013 266 640
- US-A1- 2013 266 640

### SEQUENCE LISTING

<110> SHIRE HUMAN GENETIC THERAPIES, INC.
<120> MRNA THERAPY FOR TREATMENT OF OCULAR DISEASES
<130> 2006685-0854
<140>
   <141>
<150> 61/969,483
   <151> 2014-03-24
<160> 8
<170> PatentIn version 3.5
<210> 1
   <211> 1899
   <212> RNA
   <213> Artificial Sequence
<220>
   <223> Description of Artificial Sequence: Synthetic
   polynucleotide
<220>
   <221> modified_base
   <222> (1794)..(1794)
   <223> May or may not be present
<220>
   <221> modified_base
   <222> (1896)..(1896)
   <223> May or may not be present
<220>
   <223> See specification as filed for detailed description of
   substitutions and preferred embodiments
<400> 1
   ggacagaucg ccuggagacg ccauccacgc uguuuugacc uccauagaag acaccgggac      60
   cgauccagcc uccgcggccg ggaacggugc auuggaacgc ggauuccccg ugccaagagu     120
   gacucaccgu ccuugacacg auggaagaug ccaaaaacau uaagaagggc ccagcgccau     180
   ucuacccacu cgaagacggg accgccggcg agcagcugca caaagccaug aagcgcuacg     240
   cccuggugcc cggcaccauc gccuuuaccg acgcacauau cgagguggac auuaccuacg     300
   ccgaguacuu cgagaugagc guucggcugg cagaagcuau gaagcgcuau gggcugaaua     360
   caaaccaucg gaucguggug ugcagcgaga auagcuugca guucuucaug cccguguugg     420
   gugcccuguu caucggugug gcuguggccc cagcuaacga caucuacaac gagcgcgagc     480
   ugcugaacag caugggcauc agccagccca ccgucguauu cgugagcaag aaagggcugc     540
   aaaagauccu caacgugcaa aagaagcuac cgaucauaca aaagaucauc aucauggaua     600
   gcaagaccga cuaccagggc uuccaaagca uguacaccuu cgugacuucc cauuugccac 660
   ccggcuucaa cgaguacgac uucgugcccg agagcuucga ccgggacaaa accaucgccc 720
   ugaucaugaa caguaguggc aguaccggau ugcccaaggg cguagcccua ccgcaccgca 780
   ccgcuugugu ccgauucagu caugcccgcg accccaucuu cggcaaccag aucauccccg 840
   acaccgcuau ccucagcgug gugccauuuc accacggcuu cggcauguuc accacgcugg 900
   gcuacuugau cugcggcuuu cgggucgugc ucauguaccg cuucgaggag gagcuauucu 960
   ugcgcagcuu gcaagacuau aagauucaau cugcccugcu ggugcccaca cuauuuagcu 1020
   ucuucgcuaa gagcacucuc aucgacaagu acgaccuaag caacuugcac gagaucgcca 1080
   gcggcggggc gccgcucagc aaggagguag gugaggccgu ggccaaacgc uuccaccuac 1140
   caggcauccg ccagggcuac ggccugacag aaacaaccag cgccauucug aucacccccg 1200
   aaggggacga caagccuggc gcaguaggca agguggugcc cuucuucgag gcuaaggugg 1260
   uggacuugga caccgguaag acacugggug ugaaccagcg cggcgagcug ugcguccgug 1320
   gccccaugau caugagcggc uacguuaaca accccgaggc uacaaacgcu cucaucgaca 1380
   aggacggcug gcugcacagc ggcgacaucg ccuacuggga cgaggacgag cacuucuuca 1440
   ucguggaccg gcugaagagc cugaucaaau acaagggcua ccagguagcc ccagccgaac 1500
   uggagagcau ccugcugcaa caccccaaca ucuucgacgc cggggucgcc ggccugcccg 1560
   acgacgaugc cggcgagcug cccgccgcag ucgucgugcu ggaacacggu aaaaccauga 1620
   ccgagaagga gaucguggac uauguggcca gccagguuac aaccgccaag aagcugcgcg 1680
   gugguguugu guucguggac gaggugccua aaggacugac cggcaaguug gacgcccgca 1740
   agauccgcga gauucucauu aaggccaaga agggcggcaa gaucgccgug uaacgggugg 1800
   caucccugug accccucccc agugccucuc cuggcccugg aaguugccac uccagugccc 1860
   accagccuug uccuaauaaa auuaaguugc aucaaagcu 1899
<210> 2
   <211> 1485
   <212> RNA
   <213> Artificial Sequence
<220>
   <223> Description of Artificial Sequence: Synthetic
   polynucleotide
<220>
   <221> modified_base
   <222> (1380)..(1380)
   <223> May or may not be present
<220>
   <221> modified_base
   <222> (1482)..(1482)
   <223> May or may not be present
<220>
   <223> See specification as filed for detailed description of substitutions and preferred embodiments
<400> 2
   ggacagaucg ccuggagacg ccauccacgc uguuuugacc uccauagaag acaccgggac      60
   cgauccagcc uccgcggccg ggaacggugc auuggaacgc ggauuccccg ugccaagagu     120
   gacucaccgu ccuugacacg augagcagca agggcagcgu ggugcuggcc uacagcggcg     180
   gccuggacac cagcugcauc cugguguggc ugaaggagca gggcuacgac gugaucgccu     240
   accuggccaa caucggccag aaggaggacu ucgaggaggc ccgcaagaag gcccugaagc     300
   ugggcgccaa gaagguguuc aucgaggacg ugagccgcga guucguggag gaguucaucu     360
   ggcccgccau ccagagcagc gcccuguacg aggaccgcua ccugcugggc accagccugg     420
   cccgccccug caucgcccgc aagcaggugg agaucgccca gcgcgagggc gccaaguacg     480
   ugagccacgg cgccaccggc aagggcaacg accaggugcg cuucgagcug agcugcuaca     540
   gccuggcccc ccagaucaag gugaucgccc ccuggcgcau gcccgaguuc uacaaccgcu     600
   ucaagggccg caacgaccug auggaguacg ccaagcagca cggcaucccc auccccguga     660
   cccccaagaa ccccuggagc auggacgaga accugaugca caucagcuac gaggccggca     720
   uccuggagaa ccccaagaac caggcccccc ccggccugua caccaagacc caggaccccg     780
   ccaaggcccc caacaccccc gacauccugg agaucgaguu caagaagggc gugcccguga     840
   aggugaccaa cgugaaggac ggcaccaccc accagaccag ccuggagcug uucauguacc     900
   ugaacgaggu ggccggcaag cacggcgugg gccgcaucga caucguggag aaccgcuuca     960
   ucggcaugaa gagccgcggc aucuacgaga cccccgccgg caccauccug uaccacgccc    1020
   accuggacau cgaggccuuc accauggacc gcgaggugcg caagaucaag cagggccugg    1080
   gccugaaguu cgccgagcug guguacaccg gcuucuggca cagccccgag ugcgaguucg    1140
   ugcgccacug caucgccaag agccaggagc gcguggaggg caaggugcag gugagcgugc    1200
   ugaagggcca gguguacauc cugggccgcg agagcccccu gagccuguac aacgaggagc    1260
   uggugagcau gaacgugcag ggcgacuacg agcccaccga cgccaccggc uucaucaaca    1320
   ucaacagccu gcgccugaag gaguaccacc gccugcagag caaggugacc gccaagugac    1380
   ggguggcauc ccugugaccc cuccccagug ccucuccugg cccuggaagu ugccacucca    1440
   gugcccacca gccuuguccu aauaaaauua aguugcauca aagcu                    1485
<210> 3
   <211> 140
   <212> RNA
   <213> Artificial Sequence
<220>
   <223> Description of Artificial Sequence: Synthetic
   polynucleotide
<400> 3
   ggacagaucg ccuggagacg ccauccacgc uguuuugacc uccauagaag acaccgggac 60
   cgauccagcc uccgcggccg ggaacggugc auuggaacgc ggauuccccg ugccaagagu 120
   gacucaccgu ccuugacacg 140
<210> 4
   <211> 105
   <212> RNA
   <213> Artificial Sequence
<220>
   <223> Description of Artificial Sequence: Synthetic
   polynucleotide
<400> 4
   cggguggcau cccugugacc ccuccccagu gccucuccug gcccuggaag uugccacucc 60
   agugcccacc agccuugucc uaauaaaauu aaguugcauc aagcu 105
<210> 5
   <211> 105
   <212> RNA
   <213> Artificial Sequence
<220>
   <223> Description of Artificial Sequence: Synthetic
   polynucleotide
<400> 5
   ggguggcauc ccugugaccc cuccccagug ccucuccugg cccuggaagu ugccacucca 60
   gugcccacca gccuuguccu aauaaaauua aguugcauca aagcu 105
<210> 6
   <211> 500
   <212> RNA
   <213> Artificial Sequence
<220>
   <223> Description of Artificial Sequence: Synthetic
   polynucleotide
<220>
   <221> misc_feature
   <222> (1)..(500)
   <223> This sequence may encompass 10-500 nucleotides
<220>
   <223> See specification as filed for detailed description of
   substitutions and preferred embodiments
<400> 6
   aaaaaaaaaa aaaaaaaaaa aaaaaaaaaa aaaaaaaaaa aaaaaaaaaa aaaaaaaaaa     60
   aaaaaaaaaa aaaaaaaaaa aaaaaaaaaa aaaaaaaaaa aaaaaaaaaa aaaaaaaaaa    120
   aaaaaaaaaa aaaaaaaaaa aaaaaaaaaa aaaaaaaaaa aaaaaaaaaa aaaaaaaaaa    180
   aaaaaaaaaa aaaaaaaaaa aaaaaaaaaa aaaaaaaaaa aaaaaaaaaa aaaaaaaaaa    240
   aaaaaaaaaa aaaaaaaaaa aaaaaaaaaa aaaaaaaaaa aaaaaaaaaa aaaaaaaaaa    300
   aaaaaaaaaa aaaaaaaaaa aaaaaaaaaa aaaaaaaaaa aaaaaaaaaa aaaaaaaaaa    360
   aaaaaaaaaa aaaaaaaaaa aaaaaaaaaa aaaaaaaaaa aaaaaaaaaa aaaaaaaaaa    420
   aaaaaaaaaa aaaaaaaaaa aaaaaaaaaa aaaaaaaaaa aaaaaaaaaa aaaaaaaaaa    480
   aaaaaaaaaa aaaaaaaaaa                                                500
<210> 7
   <211> 200
   <212> RNA
   <213> Artificial Sequence
<220>
   <223> Description of Artificial Sequence: Synthetic
   polynucleotide
<220>
   <221> misc_feature
   <222> (1)..(200)
   <223> This sequence may encompass 10-200 nucleotides
<220>
   <223> See specification as filed for detailed description of
   substitutions and preferred embodiments
<400> 7
   cccccccccc cccccccccc cccccccccc cccccccccc cccccccccc cccccccccc     60
   cccccccccc cccccccccc cccccccccc cccccccccc cccccccccc cccccccccc    120
   cccccccccc cccccccccc cccccccccc cccccccccc cccccccccc cccccccccc    180
   cccccccccc cccccccccc                                                200
<210> 8
   <211> 250
   <212> RNA
   <213> Artificial Sequence
<220>
   <223> Description of Artificial Sequence: Synthetic
   polynucleotide
<400> 8
   aaaaaaaaaa aaaaaaaaaa aaaaaaaaaa aaaaaaaaaa aaaaaaaaaa aaaaaaaaaa      60
   aaaaaaaaaa aaaaaaaaaa aaaaaaaaaa aaaaaaaaaa aaaaaaaaaa aaaaaaaaaa     120
   aaaaaaaaaa aaaaaaaaaa aaaaaaaaaa aaaaaaaaaa aaaaaaaaaa aaaaaaaaaa     180
   aaaaaaaaaa aaaaaaaaaa aaaaaaaaaa aaaaaaaaaa aaaaaaaaaa aaaaaaaaaa     240
   aaaaaaaaaa                                                            250

## Description

### BACKGROUND

Effective therapies are still needed for the treatment of ocular diseases, disorders or conditions such as those directly or indirectly resulting from the loss, aberrant expression, dysregulation or over-production of a ocular cellular protein. Several hurdles exist in implementing an effective treatment strategy for ocular diseases and disorders, mainly due to the unique anatomy and physiology of the eye. The combination of static barriers such as different layers and regions of the eye, and dynamic barriers such as blood flow, lymphatic clearance and tear dilution pose a significant challenge for drug delivery.

WO 2013/149140 A1 suggests intravitreal delivery, but not in the context of treating ocular diseases. US 2013/266640 A1 suggests intravitreal delivery of mRNA encoding a reporter gene; the provided example is entirely prophetic. WO 2006/110813 A2 describes the use of siRNA for use in treating ocular diseases.

Examples 43D and 84 of WO 2013/151671 A1 make reference to intravitreal delivery of mRNA. Example 84 describes intravitreal administration of saline-formulated mCherry and luciferase mRNAs and discloses that positive expression of saline-formulated mCherry mRNA was observed by fluorescence microscopy. Example 43D suggests intravitreal administration of mCherry mRNA lipoplexed with RNAIMAX™ without providing any experimental data.

### SUMMARY OF THE INVENTION

The present disclosure provides, among other things, effective methods and compositions for the treatment of ocular diseases, disorders or conditions based on messenger RNA (mRNA) therapy. The claimed invention is, in part, based on the unexpected observation that mRNA may be effectively delivered to the eye despite the uniquely challenging astatic and dynamic barriers due to uniquely complicated eye anatomy and physiology. As described herein including in the examples, the present inventors have successfully delivered mRNA encoding either fire fly luciferase (FFL) or human argininosuccinate synthetase (a protein naturally expressed in the eyes), resulting in robust protein expression throughout the eye. Therefore, the present inventors have demonstrated, *for the first time,* that mRNA based delivery may be used to effectively deliver therapeutic proteins in the eye, providing an effective yet unexpected solution for this difficult and long-standing problem of ocular drug delivery.

Thus, the invention relates to a composition comprising an mRNA encoding a therapeutic protein for use in treating an eye disease, disorder or condition in a subject in need thereof, wherein the composition is administered into an eye of the subject via intravitreal administration such that the administration of the composition results in expression and/or activity of the therapeutic protein encoded by the mRNA in the eye, wherein the mRNA is codon-optimized, has a length of 0.5 kb to 5 kb and is encapsulated within a liposome, wherein the liposome comprises one or more cationic lipids, one or more non-cationic lipids, one or more cholesterol-based lipids and one or more PEG-modified lipids. In some embodiments, the therapeutic protein encoded by the mRNA normally functions in the eye, e.g. is naturally expressed in the eyes of healthy subjects. In other embodiments, the therapeutic protein encoded by the mRNA is an antibody (e.g. anti-VEGF antibody, anti-TNFa antibody, anti-IL-6 antibody, anti-ICAM-1 antibody, or anti-VCAM-1 antibody) or a soluble receptor (e.g. soluble VEGF receptor).

Intravitreal administration is used where it is desired that expression of the mRNA is restricted to the eye, e.g. where the mRNA encodes a protein that is expressed solely in the eye or where the mRNA encodes a therapeutic protein whose activity is particularly restricted to the eye (e.g. where the protein encodes a VEGF antagonist such as an anti-VEGF antibody or a soluble VEGF receptor). In some embodiments, the expression and/or activity of the protein is detected in corneal cells, scleral cells, choroid plexus epithelial cells, ciliary body cells, retinal cells, and/or vitreous humour. In some embodiments, the expression and/or activity of the protein is detected by blood sampling. In some embodiments, the expression and/or activity of the protein is detected by sampling a vitreous humor. In some embodiments, the expression and/or activity of the protein is detected in retinal cells. In some embodiments, the expression and/or activity of the protein is detectable about 6 hours, 12 hours, 18 hours, 24 hours, 2 days, 3 days, 4 days, 5 days, 6 days, 1 week, 2 weeks, 3 weeks, 4 weeks, 1 month, 2 months, 3 months, 4 months, 5 months, 6 months or longer post-administration. For example, expression and/or activity of the protein encoded by an mRNA can be detected for at least 24 hours post administration.

In some embodiments, the mRNA in the composition of the invention will have a length of about 1 kb to about 3 kb. In some embodiments, the protein encoded by the mRNA normally functions in the eye. In some embodiments, the protein encoded by the mRNA is an antibody. In some embodiments, the antibody encoded by the mRNA is an anti-VEGF antibody, anti-TNFa antibody, anti-IL-6 antibody, anti-ICAM-1 antibody, anti-VCAM-1, or a soluble VEGF receptor (e.g., VEGFR1).

In some embodiments, the eye disease, disorder or condition is selected from AMD, PU, BRVO, CRVO, DME, CME, UME, CMV retinitis, endophthalmitis, inflammation, glaucoma, macular degeneration, scleritis, choriotetinitis, Dry eye syndrome, Stargardt disease, Norris disease, Coat's disease, persistent hyperplastic primary vitreous, familial exudative vitreoretinopathy, Leber congenital amaurosis, Retinitis Pigmentosa, X-linked retinoschisis, Leber's hereditary optic neurophathy (LHON), and/or uveitis.

In some embodiments, the composition is administered once daily, once every other day, once every three days, twice a week, once a week, once every two weeks, once every three weeks, once every four weeks, twice a month, once a month, once every two months, once every three months, once every four months, once every five months, once every six months, twice a year, or once a year. In certain embodiments, the composition is administered once a week. In certain embodiments, the composition is administered twice a month. In certain embodiments, the composition is administered once a month. In some embodiments, the composition is administered every two months.

In some embodiments, the one or more cationic lipids are selected from the group consisting of C12-200, MC3, DLinDMA, DLinkC2DMA, cKK-E12, ICE (Imidazol-based), HGT5000, HGT5001, DODAC, DDAB, DMRIE, DOSPA, DOGS, DODAP, DODMA and DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, HGT4003, and combinations thereof. Useful liposome compositions include those comprising or consisting of the following lipid combinations: (i) cKK-E12, DOPE, cholesterol and DMG-PEG2K; (ii) C12-200, DOPE, cholesterol and DMG-PEG2K. As exemplified below, lipid nanoparticles containing cKK-E12 or C12-200 as cationic component are particularly useful to achieve high expression of encapsulated mRNA in the eye.

In some embodiments, a cationic lipid suitable for the present invention has a structure of:

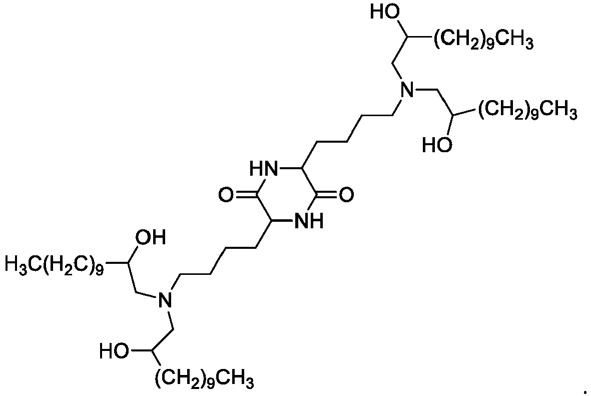

In some embodiments, suitable non-cationic lipids are selected from DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), and DOPG (,2-dioleoyl-*sn*-glycero-3-phospho-(1'-*rac*-glycerol)).

In some embodiments, a suitable cholesterol-based lipid is cholesterol or PEGylated cholesterol.

In some embodiments, one or more PEG-modified lipids suitable for the present invention is a poly(ethylene) glycol chain of up to 5 kDa in length covalently attached to a lipid with alkyl chain(s) of C₆-C₂₀ length.

In some embodiments, the cationic lipid constitutes about 30-70 % (e.g., about 30-60%, about 30-50%, about 30-40%, about 40-70%, bout 40-60%, about 40-50%, about 50-70%, or about 50-60%) of the liposome by molar ratio.

In some embodiments, the liposome comprises cKK-E12, DOPE, cholesterol, and DMG-PEG2K. In some embodiments, the liposome comprises cKK-E12, DOPE, cholesterol, and DMG-PEG2K at a ratio of 40:30:25:5, 50:25:20:5, 40:30:20:10, or 40:32:20:8. In some embodiments, the liposome comprises cKK-E12, DSPC, cholesterol, and DMG-PEG2K. In some embodiments, the liposome comprises cKK-E12, DSPC, cholesterol, and DMG-PEG2K at a ratio of 40:30:25:5, 50:25:20:5, 50:27:20:3, 40:30:20:10, 40:32:20:8, 40:32:25:3 or 40:33:25:2. In some embodiments, the liposome comprises C12-200, DOPE, cholesterol, and DMG-PEG2K. In some embodiments, the liposome comprises C12-200, DOPE, cholesterol, and DMG-PEG2K at a ratio of 50:25:20:5, 50:20:25:5, 50:27:20:3, 40:30:20:10, 40:30:25:5 or 40:32:20:8, 40:32:25:3 or 40:33:25:2..

In some embodiments, the liposome has a size of or less than about 60 nm (e.g., of or less than about 55 nm, of or less than about 50 nm, of or less than about 45 nm, of or less than about 40 nm, of or less than about 35 nm, of or less than about 30 nm, or of or less than about 25 nm). Suitable liposomes may be in the range of 25 nm to 60 nm, e.g. 30 nm to 50 nm.

In some embodiments, the mRNA comprises one or more modified nucleotides. In some embodiments, the one or more modified nucleotides comprise pseudouridine, N-1-methyl-pseudouridine, 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and/or 2-thiocytidine. In some embodiments, the mRNA is unmodified.

Other features, objects, and advantages of the present invention are apparent in the detailed description and drawings that follow. It should be understood that the detailed description and the drawings are given by way of illustration only, not limitation. Various changes and modifications within the scope of the claimed invention will become apparent to those skilled in the art.

### BRIEF DESCRIPTION OF THE DRAWING

The drawings are for illustration purposes only not for limitation.

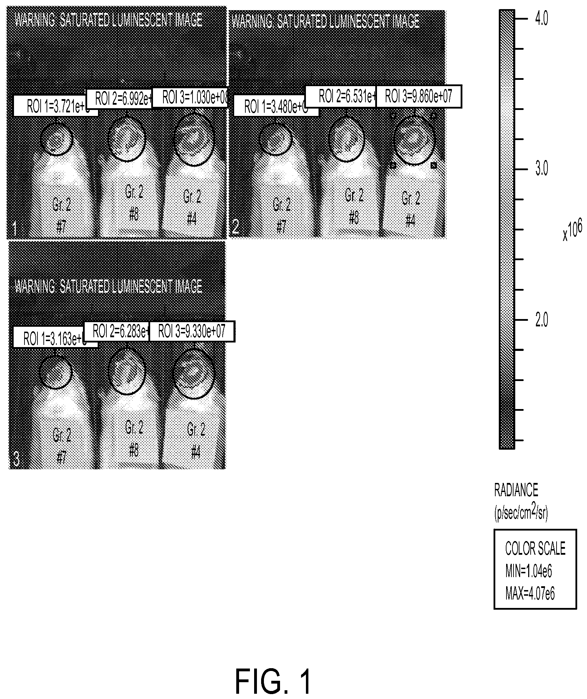

**Figure 1** depicts exemplary visualization of firefly luciferase luminescence as measured via IVIS imaging. The protein detected is a result of its production from FFL mRNA delivered via intravitreal administration using cKK-E12-based lipid nanoparticles (5.0 micrograms, based on encapsulated mRNA).

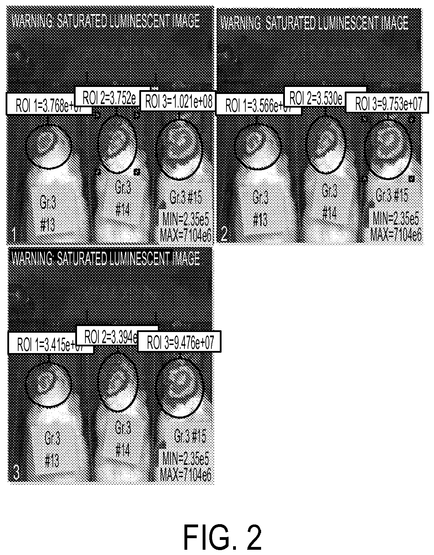

**Figure 2** depicts exemplary visualization of firefly luciferase luminescence in wild type mice as measured via IVIS imaging. The protein detected is a result of its production from FFL mRNA delivered via intravitreal administration using C12-200-based lipid nanoparticles (5.0 micrograms, based on encapsulated mRNA).

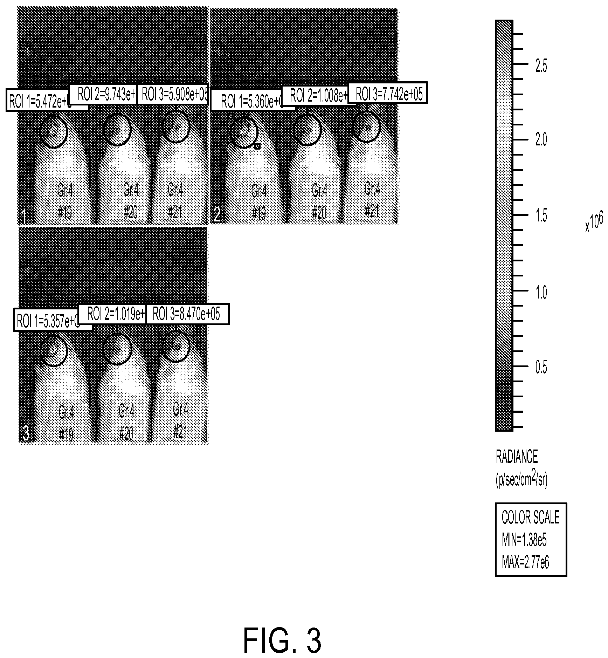

**Figure 3** depicts exemplary visualization of firefly luciferase luminescence in wild type mice as measured via IVIS imaging. The protein detected is a result of its production from FFL mRNA delivered via intravitreal administration using 25kDa branched PEI-based lipid nanoparticles (2.5 micrograms, based on encapsulated mRNA).

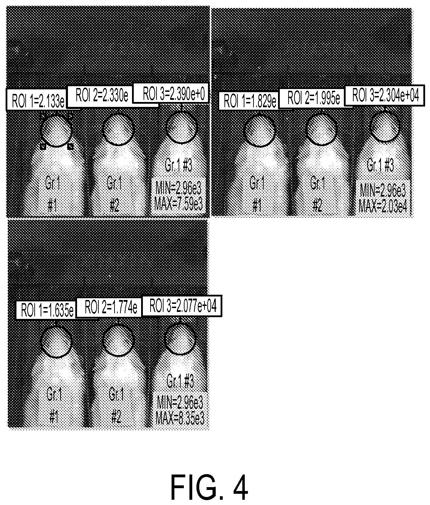

**Figure 4** depicts exemplary visualization of firefly luciferase luminescence in wild type mice as measured via IVIS imaging. A control saline injection is represented in this figure demonstrating no background luminescence.

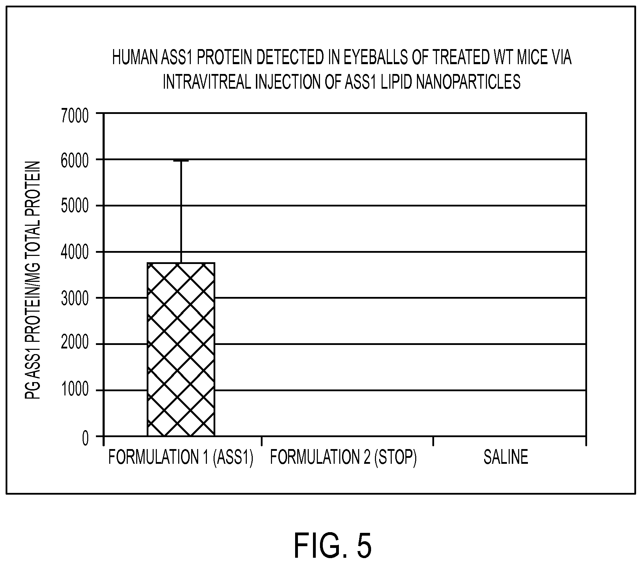

**Figure 5** depicts an exemplary graph of human argininosuccinate synthetase (ASS1) protein levels as measured via ELISA. The protein detected is a result of its production from ASS1 mRNA encapsulated in cKK-E12-based lipid nanoparticles, which was delivered by intravitreal administration to wild type mice. Eyeballs of treated mice were harvested 24 hours post-administration.

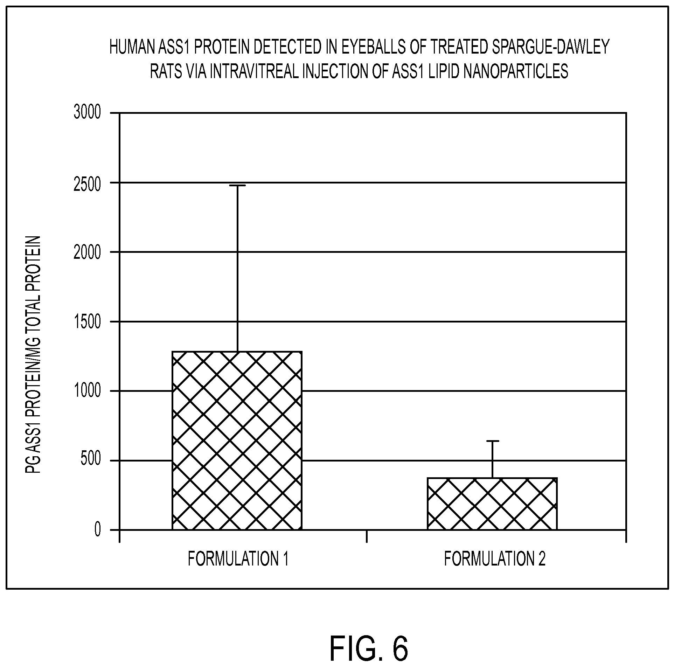

**Figure 6** depicts an exemplary graph of human argininosuccinate synthetase (ASS1) protein levels as measured via ELISA. The protein detected is a result of its production from ASS1 mRNA encapsulated in lipid nanoparticles which was delivered by intravitreal administration to Sprague-Dawley rats. Eyeballs of treated rats were harvested 24 hours post-administration. Formulations 1 and 2 represent cKK-E12-based lipid nanoparticle formulations using 5% and 3% DMG-PEG2K, respectively.

### DEFINITIONS

In order for the present invention to be more readily understood, certain terms are first defined below. Additional definitions for the following terms and other terms are set forth throughout the specification.

*Alkyl:* As used herein, "alkyl" refers to a radical of a straight-chain or branched saturated hydrocarbon group having from 1 to 15 carbon atoms ("C₁₋₁₅ alkyl"). In some embodiments, an alkyl group has 1 to 3 carbon atoms ("C₁₋₃ alkyl"). Examples of C₁₋₃ alkyl groups include methyl (C₁), ethyl (C₂), n-propyl (C₃), and isopropyl (C₃). In some embodiments, an alkyl group has 8 to 12 carbon atoms ("C₈₋₁₂ alkyl"). Examples of C₈₋₁₂ alkyl groups include, without limitation, *n*-octyl (Cs), *n*-nonyl (C₉), *n*-decyl (C₁₀), *n*-undecyl (C₁₁), *n*-dodecyl (C₁₂) and the like. The prefix "*n*-" (normal) refers to unbranched alkyl groups. For example, *n*-C₈ alkyl refers to -(CH₂)₇CH₃, *n*-C₁₀ alkyl refers to -(CH₂)₉CH₃, etc.

*Amelioration:* As used herein, the term "amelioration" is meant the prevention, reduction or palliation of a state, or improvement of the state of a subject. Amelioration includes, but does not require complete recovery or complete prevention of a disease condition. In some embodiments, amelioration includes increasing levels of relevant protein or its activity that is deficient in relevant disease tissues.

*Amino acid:* As used herein, term "amino acid," in its broadest sense, refers to any compound and/or substance that can be incorporated into a polypeptide chain. In some embodiments, an amino acid has the general structure H₂N-C(H)(R)-COOH. In some embodiments, an amino acid is a naturally occurring amino acid. In some embodiments, an amino acid is a synthetic amino acid; in some embodiments, an amino acid is a d-amino acid; in some embodiments, an amino acid is an l-amino acid. "Standard amino acid" refers to any of the twenty standard l-amino acids commonly found in naturally occurring peptides. "Nonstandard amino acid" refers to any amino acid, other than the standard amino acids, regardless of whether it is prepared synthetically or obtained from a natural source. As used herein, "synthetic amino acid" encompasses chemically modified amino acids, including but not limited to salts, amino acid derivatives (such as amides), and/or substitutions. Amino acids, including carboxy- and/or amino-terminal amino acids in peptides, can be modified by methylation, amidation, acetylation, protecting groups, and/or substitution with other chemical groups that can change the peptide's circulating half-life without adversely affecting their activity. Amino acids may participate in a disulfide bond. Amino acids may comprise one or posttranslational modifications, such as association with one or more chemical entities (*e.g.,* methyl groups, acetate groups, acetyl groups, phosphate groups, formyl moieties, isoprenoid groups, sulfate groups, polyethylene glycol moieties, lipid moieties, carbohydrate moieties, biotin moieties, *etc*.). The term "amino acid" is used interchangeably with "amino acid residue," and may refer to a free amino acid and/or to an amino acid residue of a peptide. It will be apparent from the context in which the term is used whether it refers to a free amino acid or a residue of a peptide.

*Animal*: As used herein, the term "animal" refers to any member of the animal kingdom. In some embodiments, "animal" refers to humans, at any stage of development. In some embodiments, "animal" refers to non-human animals, at any stage of development. In certain embodiments, the non-human animal is a mammal (*e.g.,* a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically-engineered animal, and/or a clone.

*Approximately or about:* As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Biologically active*: As used herein, the phrase "biologically active" refers to a characteristic of any agent that has activity in a biological system, and particularly in an organism. For instance, an agent that, when administered to an organism, has a biological effect on that organism, is considered to be biologically active. In particular embodiments, where a protein or polypeptide is biologically active, a portion of that protein or polypeptide that shares at least one biological activity of the protein or polypeptide is typically referred to as a "biologically active" portion.

*Delivery:* As used herein, the term "delivery" encompasses both local and systemic delivery. For example, delivery of mRNA encompasses situations in which an mRNA is delivered to a target tissue and the encoded protein is expressed and retained within the target tissue (aslo referred to as "local distribution" or "local delivery"), and situations in which an mRNA is delivered to a target tissue and the encoded protein is expressed and secreted into patient's circulation system (e.g., serum) and systematically distributed and taken up by other tissues (also referred to as "systemic distribution" or "systemic delivery).

*Expression*: As used herein, "expression" of a nucleic acid sequence refers to one or more of the following events: (1) production of an RNA template from a DNA sequence (e.g., by transcription); (2) processing of an RNA transcript (e.g., by splicing, editing, 5' cap formation, and/or 3' end formation); (3) translation of an RNA into a polypeptide or protein; and/or (4) post-translational modification of a polypeptide or protein. In this application, the terms "expression" and "production," and grammatical equivalent, are used inter-changeably.

*Fragment*: The term "fragment" as used herein refers to polypeptides and is defined as any discrete portion of a given polypeptide that is unique to or characteristic of that polypeptide. The term as used herein also refers to any discrete portion of a given polypeptide that retains at least a fraction of the activity of the full-length polypeptide. Preferably the fraction of activity retained is at least 10% of the activity of the full-length polypeptide. More preferably the fraction of activity retained is at least 20%, 30%, 40%, 50%, 60%, 70%, 80% or 90% of the activity of the full-length polypeptide. More preferably still the fraction of activity retained is at least 95%, 96%, 97%, 98% or 99% of the activity of the full-length polypeptide. Most preferably, the fraction of activity retained is 100% of the activity of the full-length polypeptide. The term as used herein also refers to any portion of a given polypeptide that includes at least an established sequence element found in the full-length polypeptide. Preferably, the sequence element spans at least 4-5, more preferably at least about 10, 15, 20, 25, 30, 35, 40, 45, 50 or more amino acids of the full-length polypeptide.

*Functional*: As used herein, a "functional" biological molecule is a biological molecule in a form in which it exhibits a property and/or activity by which it is characterized.

*Half-life:* As used herein, the term "half-life" is the time required for a quantity such as nucleic acid or protein concentration or activity to fall to half of its value as measured at the beginning of a time period.

*Improve, increase, or reduce*: As used herein, the terms "improve," "increase" or "reduce," or grammatical equivalents, indicate values that are relative to a baseline measurement, such as a measurement in the same individual prior to initiation of the treatment described herein, or a measurement in a control subject (or multiple control subject) in the absence of the treatment described herein. A "control subject" is a subject afflicted with the same form of disease as the subject being treated, who is about the same age as the subject being treated.

*In Vitro*: As used herein, the term "*in vitro*" refers to events that occur in an artificial environment, *e.g.,* in a test tube or reaction vessel, in cell culture, *etc.,* rather than within a multi-cellular organism.

*In Vivo*: As used herein, the term *"in vivo"* refers to events that occur within a multi-cellular organism, such as a human and a non-human animal. In the context of cell-based systems, the term may be used to refer to events that occur within a living cell (as opposed to, for example, *in vitro* systems).

*Isolated*: As used herein, the term "isolated" refers to a substance and/or entity that has been (1) separated from at least some of the components with which it was associated when initially produced (whether in nature and/or in an experimental setting), and/or (2) produced, prepared, and/or manufactured by the hand of man. Isolated substances and/or entities may be separated from about 10%, about 20%, about 30%, about 40%, about 50%, about 60%, about 70%, about 80%, about 90%, about 91%, about 92%, about 93%, about 94%, about 95%, about 96%, about 97%, about 98%, about 99%, or more than about 99% of the other components with which they were initially associated. In some embodiments, isolated agents are about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components. As used herein, calculation of percent purity of isolated substances and/or entities should not include excipients (e.g., buffer, solvent, water, etc.).

*messenger RNA (mRNA):* As used herein, the term "messenger RNA (mRNA)" refers to a polynucleotide that encodes at least one polypeptide. mRNA as used herein encompasses both modified and unmodified RNA. mRNA may contain one or more coding and non-coding regions. mRNA can be purified from natural sources, produced using recombinant expression systems and optionally purified, chemically synthesized, *etc.* Where appropriate, *e.g.,* in the case of chemically synthesized molecules, mRNA can comprise nucleoside analogs such as analogs having chemically modified bases or sugars, backbone modifications, *etc.* An mRNA sequence is presented in the 5' to 3' direction unless otherwise indicated. In some embodiments, an mRNA is or comprises natural nucleosides (*e.g.,* adenosine, guanosine, cytidine, uridine); nucleoside analogs (*e.g.,* 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and 2-thiocytidine); chemically modified bases; biologically modified bases (*e.g.,* methylated bases); intercalated bases; modified sugars (*e.g.,* 2'-fluororibose, ribose, 2'-deoxyribose, arabinose, and hexose); and/or modified phosphate groups (*e.g.,* phosphorothioates and 5'-*N*-phosphoramidite linkages).

*Nucleic acid:* As used herein, the term "nucleic acid," in its broadest sense, refers to any compound and/or substance that is or can be incorporated into a polynucleotide chain. In some embodiments, a nucleic acid is a compound and/or substance that is or can be incorporated into a polynucleotide chain via a phosphodiester linkage. In some embodiments, "nucleic acid" refers to individual nucleic acid residues (*e.g.,* nucleotides and/or nucleosides). In some embodiments, "nucleic acid" refers to a polynucleotide chain comprising individual nucleic acid residues. In some embodiments, "nucleic acid" encompasses RNA as well as single and/or double-stranded DNA and/or cDNA.

*Patient:* As used herein, the term "patient" or "subject" refers to any organism to which a provided composition may be administered, *e.g.,* for experimental, diagnostic, prophylactic, cosmetic, and/or therapeutic purposes. Typical patients include animals (*e.g.,* mammals such as mice, rats, rabbits, non-human primates, and/or humans). In some embodiments, a patient is a human. A human includes pre and post natal forms.

*Pharmaceutically acceptable*: The term "pharmaceutically acceptable" as used herein, refers to substances that, within the scope of sound medical judgment, are suitable for use in contact with the tissues of human beings and animals without excessive toxicity, irritation, allergic response, or other problem or complication, commensurate with a reasonable benefit/risk ratio.

*Pharmaceutically acceptable salt*: Pharmaceutically acceptable salts are well known in the art. For example, S. M. Berge et al., describes pharmaceutically acceptable salts in detail in J. Pharmaceutical Sciences (1977) 66:1-19. Pharmaceutically acceptable salts of the compounds disclosed herein include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or rnalonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2- naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Salts derived from appropriate bases include alkali metal, alkaline earth metal, ammonium and N⁺(C₁₋₄ alkyl)₄ salts. Representative alkali or alkaline earth metal salts include sodium, lithium, potassium, calcium, magnesium, and the like. Further pharmaceutically acceptable salts include, when appropriate, nontoxic ammonium. quaternary ammonium, and amine cations formed using counterions such as halide, hydroxide, carboxylate, sulfate, phosphate, nitrate, sulfonate and aryl sulfonate. Further pharmaceutically acceptable salts include salts formed from the quarternization of an amine using an appropriate electrophile, e.g., an alkyl halide, to form a quarternized alkylated amino salt.

*Subject*: As used herein, the term "subject" refers to a human or any non-human animal (e.g., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

*Substantially*: As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Therapeutically effective amount*: As used herein, the term "therapeutically effective amount" of a therapeutic agent means an amount that is sufficient, when administered to a subject suffering from or susceptible to a disease, disorder, and/or condition, to treat, diagnose, prevent, and/or delay the onset of the symptom(s) of the disease, disorder, and/or condition. It will be appreciated by those of ordinary skill in the art that a therapeutically effective amount is typically administered via a dosing regimen comprising at least one unit dose.

*Treatment*: As used herein, the term "treatment" (also "treat" or "treating") refers to any administration of a substance (e.g., provided compositions) that partially or completely alleviates, ameliorates, relives, inhibits, delays onset of, reduces severity of, and/or reduces incidence of one or more symptoms, features, and/or causes of a particular disease, disorder, and/or condition (*e.g.,* influenza). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

### DETAILED DESCRIPTION

The present disclosure provides, among other things, methods and compositions for treating ocular diseases, disorders or conditions based on mRNA therapy. In particular, the present disclosure provides methods for treating ocular diseases, disorders or conditions by administering to a subject in need of treatment a composition comprising an mRNA, such that the administration of the composition results in expression of the protein encoded by the mRNA in the eye. The invention specifically relates to a composition comprising an mRNA encoding a therapeutic protein for use in treating an eye disease, disorder or condition in a subject in need thereof, wherein the composition is administered into an eye of the subject via intravitreal administration such that the administration of the composition results in expression and/or activity of the therapeutic protein encoded by the mRNA in the eye, wherein the mRNA is codon-optimized, has a length of 0.5 kb to 5 kb and is encapsulated within a liposome, wherein the liposome comprises one or more cationic lipids, one or more non-cationic lipids, one or more cholesterol-based lipids and one or more PEG-modified lipids.

Various aspects of the invention are described in detail in the following sections. The use of sections is not meant to limit the invention. Each section can apply to any aspect of the invention. In this description, the use of "or" means "and/or" unless stated otherwise.

### Ocular diseases, disorders or conditions

The composition of the invention may be used to treat a subject who is suffering from or susceptible to an ocular disease, disorder or condition. As used herein, an "ocular disease, disorder or condition" refers to a disease, disorder or condition affecting the eye and/or vision. Ocular diseases, disorders or conditions can affect one or more of the following parts of the eye: eyelid, lacrimal system and orbit; conjunctiva; sclera, cornea, iris and cilliary body; lens; choroid and retina; vitreous body and globe; optic nerve and visual pathways; and ocular muscles. In some embodiments, an ocular disease, disorder or condition may be caused by a protein deficiency or dysfunctions in the eye or parts of the anatomy associated with vision. In some embodiments, an ocular disease, disorder or condition may be caused by a protein surplus, over expression, and/or over activation in the eye or parts of the anatomy associated with vision.

Exemplary ocular diseases, disorders or conditions include, but are not limited to, age-related macular degeneration (AMD), pigmentary uveitis (PU), branch retinal vein occlusion (BRVO), central retinal vein occlusion (CRVO), diabetic macular edema (DME), cystoid macular edema (CME), uveitic macular edema (UME), cytomegalovirus (CMV) retinitis, endophthalmitis, inflammation, glaucoma, macular degeneration, scleritis, choriotetinitis, and uveitis.

In various embodiments, the composition of the invention may be used to deliver an mRNA encoding a protein that is deficient in any of the ocular diseases, disorders or conditions described herein. In such embodiments, the delivery of mRNA typically results in increased protein expression and/or activity in the eye sufficient to treat protein deficiency. In some embodiments, an mRNA suitable for the invention may encode a wild-type or naturally occurring protein sequence. In some embodiments, an mRNA suitable for the invention may be a wild-type or naturally occurring sequence. In some embodiments, an mRNA suitable for the invention may encode an amino acid sequence having substantial homology or identity to the wild-type or naturally-occurring amino acid protein sequence (e.g., having at least 50%, 55%, 60%, 65%, 70%, 75%, 80%, 85%, 90%, 95%, 98% sequence identity to the wild-type or naturally-occurring sequence). In particular embodiments, sequence identity to the wild-type or naturally-occurring sequence will be in the range of 85-98%, e.g. 90-98% or 95-98%.

Mutations in more than 40 genes result in Retinitis pigmentosa (RP). For example, mutations in the ABCA4 gene are associated with Stargardt's disease, a Retinoschisin gene is mutated in Hereditary retinoschisis, the RPE65 gene is mutated in Leber's congenital amaurosis (LCA), Mitochondrial DNA mutations in ND1, ND4 or ND6 genes are found in Leber's hereditary optic neuropathy and the Myo7 gene is mutated in Usher disease.

In some embodiments, the present invention may be used to deliver an mRNA encoding a therapeutic agent that inhibits, down-regulates, reduces a protein expression and/or activity, the excess level of which is associated with an ocular disease, disorder or condition. Such a therapeutic agent may be a peptide, an antibody or other polypeptides or proteins.

In some embodiments, the present invention may be used to deliver an mRNA encoding an antibody, a soluble receptor or other binding protein. Typically, a suitable mRNA encodes an antibody inhibits, down-regulates, or reduces a protein that is present in excess in amount and/or activity in an ocular disease, disorder or condition. In some embodiments, a suitable mRNA encodes an antibody activates, up-regulates or increases a protein activity that is deficient in an ocular disease, disorder or condition. Suitable exemplary antibodies encoded by mRNAs according to the present invention include, but are not limited to, antibodies against VEGF, TNFα, IL-6, ICAM-1, VCAM-1, or soluble receptors such as VEGF receptors (e.g., VEGFR1).

As used herein, the term "antibody" encompasses both intact antibody and antibody fragment. Typically, an intact "antibody" is an immunoglobulin that binds specifically to a particular antigen. An antibody may be a member of any immunoglobulin class, including any of the human classes: IgG, IgM, IgE, IgA, and IgD. Typically, an intact antibody is a tetramer. Each tetramer is composed of two identical pairs of polypeptide chains, each pair having one "light" (approximately 25 kD) and one "heavy" chain (approximately 50-70 kD). The N-terminus of each chain defines a variable region of about 100 to 110 or more amino acids primarily responsible for antigen recognition. The terms "variable light chain"(VL) and "variable heavy chain" (VH) refer to these corresponding regions on the light and heavy chain respectively. Each variable region can be further subdivided into hypervariable (HV) and framework (FR) regions. The hypervariable regions comprise three areas of hypervariability sequence called complementarity determining regions (CDR 1, CDR 2 and CDR 3), separated by four framework regions (FR1, FR2, FR2, and FR4) which form a beta-sheet structure and serve as a scaffold to hold the HV regions in position. The C-terminus of each heavy and light chain defines a constant region consisting of one domain for the light chain (CL) and three for the heavy chain (CH1, CH2 and CH3). A light chain of immunoglobulins can be further differentiated into the isotypes *kappa* and *lamda.*

In some embodiments, the terms "intact antibody" or "fully assembled antibody" are used in reference to an antibody that contains two heavy chains and two light chains, optionally associated by disulfide bonds as occurs with naturally-produced antibodies. In some embodiments, an antibody according to the present invention is an antibody fragment.

In some embodiments, the present invention can be used to deliver an "antibody fragment." As used herein, an "antibody fragment" includes a portion of an intact antibody, such as, for example, the antigen-binding or variable region of an antibody. Examples of antibody fragments include Fab, Fab', F(ab')₂, and Fv fragments; triabodies; tetrabodies; linear antibodies; single-chain antibody molecules; and multi specific antibodies formed from antibody fragments. For example, antibody fragments include isolated fragments, "Fv" fragments, consisting of the variable regions of the heavy and light chains, recombinant single chain polypeptide molecules in which light and heavy chain variable regions are connected by a peptide linker ("ScFv proteins"), and minimal recognition units consisting of the amino acid residues that mimic the hypervariable region. In many embodiments, an antibody fragment contains a sufficient sequence of the parent antibody of which it is a fragment that it binds to the same antigen as does the parent antibody; in some embodiments, a fragment binds to the antigen with a comparable affinity to that of the parent antibody and/or competes with the parent antibody for binding to the antigen. Examples of antigen binding fragments of an antibody include, but are not limited to, Fab fragment, Fab' fragment, F(ab')₂ fragment, scFv fragment, Fv fragment, dsFv diabody, dAb fragment, Fd' fragment, Fd fragment, and an isolated complementarity determining region (CDR). Suitable antibodies include monoclonal antibodies, polyclonal antibodies, antibody mixtures or cocktails, human or humanized antibodies, chimeric antibodies, or bi-specific antibodies.

### mRNA Synthesis

mRNAs according to the present invention may be synthesized according to any of a variety of known methods. For example, mRNAs according to the present invention may be synthesized via *in vitro* transcription (IVT). Briefly, IVT is typically performed with a linear or circular DNA template containing a promoter, a pool of ribonucleotide triphosphates, a buffer system that may include DTT and magnesium ions, and an appropriate RNA polymerase (e.g., T3, T7 or SP6 RNA polymerase), DNAse I, pyrophosphatase, and/or RNAse inhibitor. The exact conditions will vary according to the specific application.

In some embodiments, for the preparation of mRNA according to the invention, a DNA template is transcribed *in vitro.* A suitable DNA template typically has a promoter, for example a T3, T7 or SP6 promoter, for *in vitro* transcription, followed by desired nucleotide sequence for desired mRNA and a termination signal.

Desired mRNA sequence(s) according to the invention may be determined and incorporated into a DNA template using standard methods. For example, starting from a desired amino acid sequence (e.g., an enzyme sequence), a virtual reverse translation is carried out based on the degenerated genetic code. Optimization algorithms may then be used for selection of suitable codons. Typically, the G/C content can be optimized to achieve the highest possible G/C content on one hand, taking into the best possible account the frequency of the tRNAs according to codon usage on the other hand. The optimized RNA sequence can be established and displayed, for example, with the aid of an appropriate display device and compared with the original (wild-type) sequence. A secondary structure can also be analyzed to calculate stabilizing and destabilizing properties or, respectively, regions of the RNA. Codon-optimization of the mRNA is performed to optimize expression in target cells. For example, if the mRNA is for delivery to a human subject, the mRNA will be codon-optimized for expression in human cells.

### Modified mRNA

In some embodiments, mRNA according to the present invention may be synthesized as unmodified or modified mRNA. Typically, mRNAs are modified to enhance stability. Modifications of mRNA can include, for example, modifications of the nucleotides of the RNA. An modified mRNA according to the invention can thus include, for example, backbone modifications, sugar modifications or base modifications. In some embodiments, mRNAs may be synthesized from naturally occurring nucleotides and/or nucleotide analogues (modified nucleotides) including, but not limited to, purines (adenine (A), guanine (G)) or pyrimidines (thymine (T), cytosine (C), uracil (U)), and as modified nucleotides analogues or derivatives of purines and pyrimidines, such as e.g. 1-methyl-adenine, 2-methyl-adenine, 2-methylthio-N-6-isopentenyl-adenine, N6-methyl-adenine, N6-isopentenyl-adenine, 2-thio-cytosine, 3-methyl-cytosine, 4-acetyl-cytosine, 5-methyl-cytosine, 2,6-diaminopurine, 1-methyl-guanine, 2-methyl-guanine, 2,2-dimethyl-guanine, 7-methyl-guanine, inosine, 1-methyl-inosine, pseudouracil (5-uracil), dihydro-uracil, 2-thio-uracil, 4-thio-uracil, 5-carboxymethylaminomethyl-2-thio-uracil, 5-(carboxyhydroxymethyl)-uracil, 5-fluoro-uracil, 5-bromo-uracil, 5-carboxymethylaminomethyl-uracil, 5-methyl-2-thio-uracil, 5-methyl-uracil, N-uracil-5-oxyacetic acid methyl ester, 5-methylaminomethyl-uracil, 5-methoxyaminomethyl-2-thio-uracil, 5'-methoxycarbonylmethyl-uracil, 5-methoxy-uracil, uracil-5-oxyacetic acid methyl ester, uracil-5-oxyacetic acid (v), 1-methyl-pseudouracil, queosine, beta-D-mannosyl-queosine, wybutoxosine, and phosphoramidates, phosphorothioates, peptide nucleotides, methylphosphonates, 7-deazaguanosine, 5-methylcytosine and inosine. The preparation of such analogues is known to a person skilled in the art e.g. from the U.S. Pat. No. 4,373,071, U.S. Pat. No. 4,401,796, U.S. Pat. No. 4,415,732, U.S. Pat. No. 4,458,066, U.S. Pat. No. 4,500,707, U.S. Pat. No. 4,668,777, U.S. Pat. No. 4,973,679, U.S. Pat. No. 5,047,524, U.S. Pat. No. 5,132,418, U.S. Pat. No. 5,153,319, U.S. Pat. Nos. 5,262,530 and 5,700,642.

In some embodiments, mRNAs may contain RNA backbone modifications. Typically, a backbone modification is a modification in which the phosphates of the backbone of the nucleotides contained in the RNA are modified chemically. Exemplary backbone modifications typically include, but are not limited to, modifications from the group consisting of methylphosphonates, methylphosphoramidates, phosphoramidates, phosphorothioates (e.g. cytidine 5'-O-(1-thiophosphate)), boranophosphates, positively charged guanidinium groups etc., which means by replacing the phosphodiester linkage by other anionic, cationic or neutral groups.

In some embodiments, mRNAs may contain sugar modifications. A typical sugar modification is a chemical modification of the sugar of the nucleotides it contains including, but not limited to, sugar modifications chosen from the group consisting of 2'-deoxy-2'-fluoro-oligoribonucleotide (2'-fluoro-2'-deoxycytidine 5'-triphosphate, 2'-fluoro-2'-deoxyuridine 5'-triphosphate), 2'-deoxy-2'-deamine-oligoribonucleotide (2'-amino-2'-deoxycytidine 5'-triphosphate, 2'-amino-2'-deoxyuridine 5'-triphosphate), 2'-O-alkyloligoribonucleotide, 2'-deoxy-2'-C-alkyloligoribonucleotide (2'-O-methylcytidine 5'-triphosphate, 2'-methyluridine 5'-triphosphate), 2'-C-alkyloligoribonucleotide, and isomers thereof (2'-aracytidine 5'-triphosphate, 2'-arauridine 5'-triphosphate), or azidotriphosphates (2'-azido-2'-deoxycytidine 5'-triphosphate, 2'-azido-2'-deoxyuridine 5'-triphosphate).

In some embodiments, mRNAs may contain modifications of the bases of the nucleotides (base modifications). A modified nucleotide which contains a base modification is also called a base-modified nucleotide. Examples of such base-modified nucleotides include, but are not limited to, 2-amino-6-chloropurine riboside 5'-triphosphate, 2-aminoadenosine 5'-triphosphate, 2-thiocytidine 5'-triphosphate, 2-thiouridine 5'-triphosphate, 4-thiouridine 5'-triphosphate, 5-aminoallylcytidine 5'-triphosphate, 5-aminoallyluridine 5'-triphosphate, 5-bromocytidine 5'-triphosphate, 5-bromouridine 5'-triphosphate, 5-iodocytidine 5'-triphosphate, 5-iodouridine 5'-triphosphate, 5-methylcytidine 5'-triphosphate, 5-methyluridine 5'-triphosphate, 6-azacytidine 5'-triphosphate, 6-azauridine 5'-triphosphate, 6-chloropurine riboside 5'-triphosphate, 7-deazaadenosine 5'-triphosphate, 7-deazaguanosine 5'-triphosphate, 8-azaadenosine 5'-triphosphate, 8-azidoadenosine 5'-triphosphate, benzimidazole riboside 5'-triphosphate, N1-methyladenosine 5'-triphosphate, N1-methylguanosine 5'-triphosphate, N6-methyladenosine 5'-triphosphate, O6-methylguanosine 5'-triphosphate, pseudouridine 5'-triphosphate, puromycin 5'-triphosphate or xanthosine 5'-triphosphate.

Typically, mRNA synthesis includes the addition of a "cap" on the N-terminal (5') end, and a "tail" on the C-terminal (3') end. The presence of the cap is important in providing resistance to nucleases found in most eukaryotic cells. The presence of a "tail" serves to protect the mRNA from exonuclease degradation.

### Cap structure

In some embodiments, mRNAs include a 5' cap structure. A 5' cap is typically added as follows: first, an RNA terminal phosphatase removes one of the terminal phosphate groups from the 5' nucleotide, leaving two terminal phosphates; guanosine triphosphate (GTP) is then added to the terminal phosphates via a guanylyl transferase, producing a 5'5'5 triphosphate linkage; and the 7-nitrogen of guanine is then methylated by a methyltransferase. Examples of cap structures include, but are not limited to, m7G(5')ppp (5'(A,G(5')ppp(5')A and G(5')ppp(5')G.

Naturally occurring cap structures comprise a 7-methyl guanosine that is linked via a triphosphate bridge to the 5'-end of the first transcribed nucleotide, resulting in a dinucleotide cap of m⁷G(5')ppp(5')N, where N is any nucleoside. *In vivo,* the cap is added enzymatically. The cap is added in the nucleus and is catalyzed by the enzyme guanylyl transferase. The addition of the cap to the 5' terminal end of RNA occurs immediately after initiation of transcription. The terminal nucleoside is typically a guanosine, and is in the reverse orientation to all the other nucleotides, i.e., G(5')ppp(5')GpNpNp.

A common cap for mRNA produced by *in vitro* transcription is m⁷G(5')ppp(5')G, which has been used as the dinucleotide cap in transcription with T7 or SP6 RNA polymerase *in vitro* to obtain RNAs having a cap structure in their 5'-termini. The prevailing method for the *in vitro* synthesis of capped mRNA employs a pre-formed dinucleotide of the form m⁷G(5')ppp(5')G ("m⁷GpppG") as an initiator of transcription.

To date, a usual form of a synthetic dinucleotide cap used in *in vitro* translation experiments is the Anti-Reverse Cap Analog ("ARCA") or modified ARCA, which is generally a modified cap analog in which the 2' or 3' OH group is replaced with -OCH₃.

Additional cap analogs include, but are not limited to, a chemical structures selected from the group consisting of m⁷GpppG, m⁷GpppA, m⁷GpppC; unmethylated cap analogs (e.g., GpppG); dimethylated cap analog (e.g., m^{2,7}GpppG), trimethylated cap analog (e.g., m^{2,2,7}GpppG), dimethylated symmetrical cap analogs (e.g., m⁷Gpppm⁷G), or anti reverse cap analogs (e.g., ARCA; m^{7,2'Ome}GpppG, m^{72'd}GpppG, m^{7,3'Ome}GpppG, m^{7,3'd} GpppG and their tetraphosphate derivatives) (see, e.g., Jemielity, J. et al., "Novel 'anti-reverse' cap analogs with superior translational properties", RNA, 9: 1108-1122 (2003)).

In some embodiments, a suitable cap is a 7-methyl guanylate ("m⁷G") linked via a triphosphate bridge to the 5'-end of the first transcribed nucleotide, resulting in m⁷G(5')ppp(5')N, where N is any nucleoside. A preferred embodiment of a m⁷G cap utilized in embodiments of the invention is m⁷G(5')ppp(5')G.

In some embodiments, the cap is a Cap0 structure. Cap0 structures lack a 2'-O-methyl residue of the ribose attached to bases 1 and 2. In some embodiments, the cap is a Cap1 structure. Cap1 structures have a 2'-O-methyl residue at base 2. In some embodiments, the cap is a Cap2 structure. Cap2 structures have a 2'-O-methyl residue attached to both bases 2 and 3.

A variety of m⁷G cap analogs are known in the art, many of which are commercially available. These include the m⁷GpppG described above, as well as the ARCA 3'-OCH₃ and 2'-OCH₃ cap analogs (Jemielity, J. et al., RNA, 9: 1108-1122 (2003)). Additional cap analogs for use in embodiments of the invention include N7-benzylated dinucleoside tetraphosphate analogs (described in Grudzien, E. et al., RNA, 10: 1479-1487 (2004)), phosphorothioate cap analogs (described in Grudzien-Nogalska, E., et al., RNA, 13: 1745-1755 (2007)), and cap analogs (including biotinylated cap analogs) described in U.S. Patent Nos. 8,093,367 and 8,304,529.

### Tail structure

Typically, the presence of a "tail" serves to protect the mRNA from exonuclease degradation. The poly A tail is thought to stabilize natural messengers and synthetic sense RNA. Therefore, in certain embodiments a long poly A tail can be added to an mRNA molecule thus rendering the RNA more stable. Poly A tails can be added using a variety of art-recognized techniques. For example, long poly A tails can be added to synthetic or *in vitro* transcribed RNA using poly A polymerase (Yokoe, et al. Nature Biotechnology. 1996; 14: 1252-1256). A transcription vector can also encode long poly A tails. In addition, poly A tails can be added by transcription directly from PCR products. Poly A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1991 edition)).

In some embodiments, mRNAs include a 3' poly(A) tail structure. Typically, the length of the poly A tail can be at least about 10, 50, 100, 200, 300, 400 at least 500 nucleotides (SEQ ID NO: 6). In some embodiments, a poly-A tail on the 3' terminus of mRNA typically includes about 10 to 300 adenosine nucleotides (e.g., about 10 to 200 adenosine nucleotides, about 10 to 150 adenosine nucleotides, about 10 to 100 adenosine nucleotides, about 20 to 70 adenosine nucleotides, or about 20 to 60 adenosine nucleotides). A poly-A tail of 10 to 100 adenosine nucleotides, for example of about 20 to 70 adenosine nucleotides, or of about 20 to 60 adenosine nucleotides, is suitable for practicing the invention. In some embodiments, a poly(U) tail may be used to instead of a poly(A) tail described herein. In some embodiments, a poly(U) tail may be added to a poly(A) tail described herein. In some embodiments, mRNAs include a 3' poly(C) tail structure. A suitable poly(C) tail on the 3' terminus of mRNA typically include about 10 to 200 cytosine nucleotides (SEQ ID NO: 7) (*e.g.,* about 10 to 150 cytosine nucleotides, about 10 to 100 cytosine nucleotides, about 20 to 70 cytosine nucleotides, about 20 to 60 cytosine nucleotides, or about 10 to 40 cytosine nucleotides). A poly-C tail of about 20 to 70 cytosine nucleotides, for example of about 20 to 60 cytosine nucleotides, or of about 10 to 40 cytosine nucleotides, is suitable for practicing the invention. The poly(C) tail may be added to a poly(A) and/or poly(U) tail or may substitute the poly(A) and/or poly(U) tail.

In some embodiments, the length of the poly(A), poly(U) or poly(C) tail is adjusted to control the stability of a modified sense mRNA molecule of the invention and, thus, the transcription of protein. For example, since the length of a tail structure can influence the half-life of a sense mRNA molecule, the length of the tail can be adjusted to modify the level of resistance of the mRNA to nucleases and thereby control the time course of polynucleotide expression and/or polypeptide production in a target cell.

### 5' and 3' Untranslated Region

In some embodiments, mRNAs include a 5' and/or 3' untranslated region. In some embodiments, a 5' untranslated region includes one or more elements that affect an mRNA's stability or translation, for example, an iron responsive element. In some embodiments, a 5' untranslated region may be between about 50 and 500 nucleotides in length.

In some embodiments, a 3' untranslated region includes one or more of a polyadenylation signal, a binding site for proteins that affect an mRNA's stability of location in a cell, or one or more binding sites for miRNAs. In some embodiments, a 3' untranslated region may be between 50 and 500 nucleotides in length or longer.

Exemplary 3' and/or 5' UTR sequences can be derived from mRNA molecules which are stable (e.g., globin, actin, GAPDH, tubulin, histone, or citric acid cycle enzymes) to increase the stability of the sense mRNA molecule. For example, a 5' UTR sequence may include a partial sequence of a CMV immediate-early 1 (IE1) gene, or a fragment thereof to improve the nuclease resistance and/or improve the half-life of the polynucleotide. Also contemplated is the inclusion of a sequence encoding human growth hormone (hGH), or a fragment thereof to the 3' end or untranslated region of the polynucleotide (e.g., mRNA) to further stabilize the polynucleotide. Generally, these modifications improve the stability and/or pharmacokinetic properties (e.g., half-life) of the polynucleotide relative to their unmodified counterparts, and include, for example modifications made to improve such polynucleotides' resistance to *in vivo* nuclease digestion.

### Delivery Vehicles

As used herein, the terms "delivery vehicle," "transfer vehicle," "nanoparticle" or grammatical equivalent, are used interchangeably.

mRNAs may be delivered via a single delivery vehicle or via one or more delivery vehicles each of a different composition. Suitable delivery vehicles include, but are not limited to polymer based carriers, such as polyethyleneimine (PEI), lipid nanoparticles and liposomes, nanoliposomes, ceramide-containing nanoliposomes, proteoliposomes, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, poly(D-arginine), sol-gels, nanodendrimers, starch-based delivery systems, micelles, emulsions, niosomes, multi-domain-block polymers (vinyl polymers, polypropyl acrylic acid polymers, dynamic polyconjugates).

### Liposomal delivery vehicles

In accordance with the invention, a suitable delivery vehicle is a liposomal delivery vehicle. As used herein, liposomal delivery vehicles are characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol., 16: 307-321, 1998). Bilayer membranes of the liposomes can also be formed by amphophilic polymers and surfactants (e.g., polymerosomes, niosomes, etc.). In the context of the present invention, a liposomal delivery vehicle serves to transport a desired mRNA to a target cell or tissue. The process of incorporation of a desired mRNA into a liposome is often referred to as "loading". Exemplary methods are described in Lasic, et al., FEBS Lett., 312: 255-258, 1992. The liposome-incorporated nucleic acids may be completely or partially located in the interior space of the liposome, within the bilayer membrane of the liposome, or associated with the exterior surface of the liposome membrane. The incorporation of a nucleic acid into liposomes is also referred to herein as "encapsulation" wherein the nucleic acid is entirely contained within the interior space of the liposome. The purpose of incorporating a mRNA into a transfer vehicle, such as a liposome, is often to protect the nucleic acid from an environment which may contain enzymes or chemicals that degrade nucleic acids and/or systems or receptors that cause the rapid excretion of the nucleic acids. Accordingly, in some embodiments, a suitable delivery vehicle is capable of enhancing the stability of the mRNA contained therein and/or facilitate the delivery of mRNA to the target cell or tissue.

### Cationic Lipids

In some embodiments, liposomes may comprise one or more cationic lipids. As used herein, the phrase "cationic lipid" refers to any of a number of lipid species that have a net positive charge at a selected pH, such as physiological pH. Several cationic lipids have been described in the literature, many of which are commercially available. Particularly suitable cationic lipids for use in the compositions of the invention include those described in international patent publications WO 2010/053572 (and particularly, CI 2-200 described at paragraph [00225]) and WO 2012/170930. In certain embodiments, the compositions of the invention employ a lipid nanoparticles comprising an ionizable cationic lipid described in U.S. provisional patent application 61/617,468, filed March 29, 2012, such as, e.g, (15Z, 18Z)-N,N-dimethyl-6-(9Z, 12Z)-octadeca-9,12-dien-1-yl)tetracosa-15,18-dien-1-amine (HGT5000), ( 15Z, 18Z)-N,N-dimethyl-6-((9Z, 12Z)-octadeca-9,12-dien-1-yl)tetracosa-4,15,18-trien-1-amine (HGT5001), and (15Z,18Z)-N,N-dimethyl-6-((9Z, 12Z)-octadeca-9, 12-dien- 1 - yl)tetracosa-5, 15 , 18-trien- 1 -amine (HGT5002).

In some embodiments, provided liposomes include a cationic lipid described in WO 2013/063468 and in U.S. provisional application entitled "Lipid Formulations for Delivery of Messernger RNA" filed concurrently with the present application on even date. In some embodiments, a cationic lipid comprises a compound of formula **I-c1-a:** 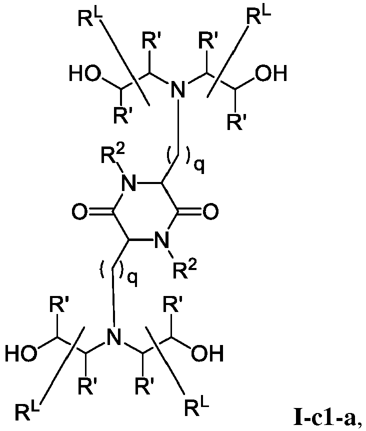 or a pharmaceutically acceptable salt thereof, wherein:
each R² independently is hydrogen or C₁₋₃ alkyl;
each q independently is 2 to 6;
each R' independently is hydrogen or C₁₋₃ alkyl;
and each R^{L} independently is C₈₋₁₂ alkyl.

In some embodiments, each R² independently is hydrogen, methyl or ethyl. In some embodiments, each R² independently is hydrogen or methyl. In some embodiments, each R² is hydrogen.

In some embodiments, each q independently is 3 to 6. In some embodiments, each q independently is 3 to 5. In some embodiments, each q is 4.

In some embodiments, each R' independently is hydrogen, methyl or ethyl. In some embodiments, each R' independently is hydrogen or methyl. In some embodiments, each R' independently is hydrogen.

In some embodiments, each R^{L} independently is C₈₋₁₂ alkyl. In some embodiments, each R^{L} independently is *n*-C₈₋₁₂ alkyl. In some embodiments, each R^{L} independently is C₉₋₁₁ alkyl. In some embodiments, each R^{L} independently is *n*-C₉₋₁₁ alkyl. In some embodiments, each R^{L} independently is C₁₀ alkyl. In some embodiments, each R^{L} independently is *n*-C₁₀ alkyl.

In some embodiments, each R² independently is hydrogen or methyl; each q independently is 3 to 5; each R' independently is hydrogen or methyl; and each R^{L} independently is C₈₋₁₂ alkyl.

In some embodiments, each R² is hydrogen; each q independently is 3 to 5; each R' is hydrogen; and each R^{L} independently is C₈₋₁₂ alkyl.

In some embodiments, each R² is hydrogen; each q is 4; each R' is hydrogen; and each R^{L} independently is C₈₋₁₂ alkyl.

In some embodiments, a cationic lipid comprises a compound of formula **I-g:** 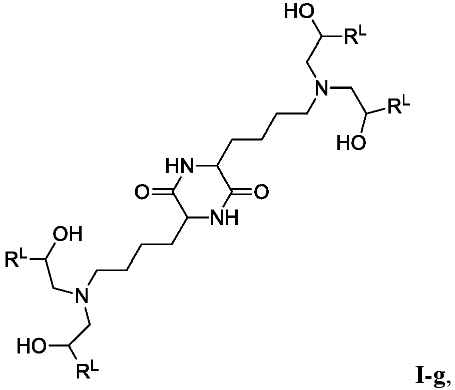 or a pharmaceutically acceptable salt thereof, wherein each R^{L} independently is C₈₋₁₂ alkyl. In some embodiments, each R^{L} independently is *n*-C₈₋₁₂ alkyl. In some embodiments, each R^{L} independently is C₉₋₁₁ alkyl. In some embodiments, each R^{L} independently is *n*-C₉₋₁₁ alkyl. In some embodiments, each R^{L} independently is C₁₀ alkyl. In some embodiments, each R^{L} is *n*-C₁₀ alkyl.

In particular embodiments, provided liposomes include a cationic lipid cKK-E12, or (3,6-bis(4-(bis(2-hydroxydodecyl)amino)butyl)piperazine-2,5-dione). Structure of cKK-E12 is shown below:

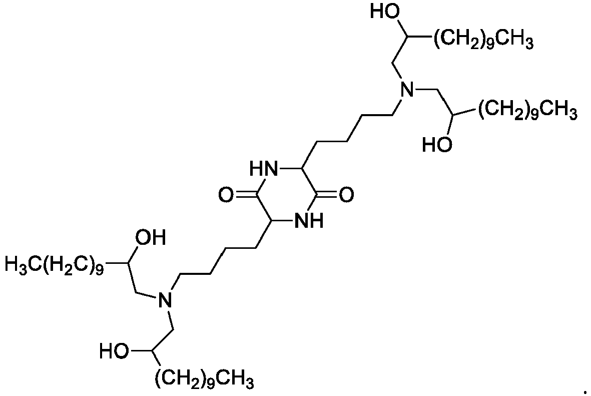

As described in the Examples section below, the present inventors observed that liposomes based on this particular class of cationic lipids, such as, those having a structure of formula **I-c1-a or** formula **I-g** described herein (e.g., cKK-E12) are unexpectedly effective in delivering mRNA and producing encoded protein *in vivo.* Although mRNA encoding PAH protein is used as an example in this application, it is contemplated that this class of cationic lipids having a structure of formula **I-c1-a or** formula **I-g** described herein (e.g., cKK-E12) can be useful in delivering any mRNA for highly efficient and sustained production of protein (e.g., therapeutic protein) *in vivo.* For example, cationic lipids having a structure of formula **I-c1-a** or formula **I-g** described herein (e.g., cKK-E12) can be used to deliver an mRNA that encodes one or more naturally occurring peptides or one or more modified or non-natural peptides. In some embodiments, cationic lipids having a structure of formula **I-c1-a** or formula **I-g** described herein (e.g., cKK-E12) can be used to deliver an mRNA that encodes an intracellular protein including, but not limited to, a cytosolic protein (e.g., a chaperone protein, an intracellular enzyme (e.g., mRNA encoding an enzyme associated with urea cycle or lysosomal storage disorders)), a protein associated with the actin cytoskeleton, a protein associated with the plasma membrane, a perinuclear protein, a nuclear protein (e.g., a transcription factor), and any other protein involved in cellular metabolism, DNA repair, transcription and/or translation). In some embodiments, cationic lipids having a structure of formula **I-c1-a or** formula **I-g** described herein (e.g., cKK-E12) can be used to deliver an mRNA that encodes a transmembrane protein, such as, an ion channel protein. In some embodiments, cationic lipids having a structure of formula **I-c1-a or** formula **I-g** described herein (e.g., cKK-E12) can be used to deliver an mRNA that encodes an extracellular protein including, but not limited to, a protein associated with the extracellular matrix, a secreted protein (e.g., hormones and/or neurotransmitters).

In some embodiments, one or more cationic lipids suitable for the present invention may be N-[1-(2,3-dioleyloxy)propyl]-N,N,N-trimethylammonium chloride or "DOTMA". (Feigner et al. (Proc. Nat'l Acad. Sci. 84, 7413 (1987); U.S. Pat. No. 4,897,355). DOTMA can be formulated alone or can be combined with the neutral lipid, dioleoylphosphatidyl-ethanolamine or "DOPE" or other cationic or non-cationic lipids into a liposomal transfer vehicle or a lipid nanoparticle, and such liposomes can be used to enhance the delivery of nucleic acids into target cells. Other suitable cationic lipids include, for example, 5-carboxyspermylglycinedioctadecylamide or "DOGS," 2,3-dioleyloxy-N-[2(spermine-carboxamido)ethyl]-N,N-dimethyl-1-propanaminium or "DOSPA" (Behr et al. Proc. Nat.'l Acad. Sci. 86, 6982 (1989); U.S. Pat. No. 5,171,678; U.S. Pat. No. 5,334,761), 1,2-Dioleoyl-3-Dimethylammonium-Propane or "DODAP", 1,2-Dioleoyl-3-Trimethylammonium-Propane or "DOTAP".

Additional exemplary cationic lipids also include 1,2-distearyloxy-N,N-dimethyl-3-aminopropane or "DSDMA", 1,2-dioleyloxy-N,N-dimethyl-3-aminopropane or "DODMA", 1 ,2-dilinoleyloxy-N,N-dimethyl-3-aminopropane or "DLinDMA", 1,2-dilinolenyloxy-N,N-dimethyl-3-aminopropane or "DLenDMA", N-dioleyl-N,N-dimethylammonium chloride or "DODAC", N,N-distearyl-N,N-dimethylarnrnonium bromide or "DDAB", N-(1,2-dimyristyloxyprop-3-yl)-N,N-dimethyl-N-hydroxyethyl ammonium bromide or "DMRIE", 3-dimethylamino-2-(cholest-5-en-3-beta-oxybutan-4-oxy)-1-(cis,cis-9,12-octadecadienoxy)propane or "CLinDMA", 2-[5'-(cholest-5-en-3-beta-oxy)-3'-oxapentoxy)-3-dimethy 1-1-(cis,cis-9',1-2'-octadecadienoxy)propane or "CpLinDMA", N,N-dimethyl-3,4-dioleyloxybenzylamine or "DMOBA", 1 ,2-N,N'-dioleylcarbamyl-3-dimethylaminopropane or "DOcarbDAP", 2,3-Dilinoleoyloxy-N,N-dimethylpropylamine or "DLinDAP", 1,2-N,N'-Dilinoleylcarbamyl-3-dimethylaminopropane or "DLincarbDAP", 1 ,2-Dilinoleoylcarbamyl-3-dimethylaminopropane or "DLinCDAP", 2,2-dilinoleyl-4-dimethylaminomethyl-[1,3]-dioxolane or "DLin- -DMA", 2,2-dilinoleyl-4-dimethylaminoethyl-[1,3]-dioxolane or "DLin-K-XTC2-DMA", and 2-(2,2-di((9Z,12Z)-octadeca-9,12-dien-1-yl)-1,3-dioxolan-4-yl)-N,N-dimethylethanamine (DLin-KC2-DMA)) (see, WO 2010/042877; Semple et al., Nature Biotech. 28: 172-176 (2010)), or mixtures thereof. (Heyes, J., et al., J Controlled Release 107: 276-287 (2005); Morrissey, DV., et al., Nat. Biotechnol. 23(8): 1003-1007 (2005); PCT Publication WO2005/121348A1). In some embodiments, one or more of the cationic lipids comprise at least one of an imidazole, dialkylamino, or guanidinium moiety.

In some embodiments, the one or more cationic lipids may be chosen from XTC (2,2-Dilinoleyl-4-dimethylaminoethyl-[1,3]-dioxolane), MC3 (((6Z,9Z,28Z,31Z)-heptatriaconta-6,9,28,31-tetraen-19-yl 4-(dimethylamino)butanoate), ALNY-100 ((3aR,5s,6aS)-N,N-dimethyl-2,2-di((9Z,12Z)-octadeca-9,12-dienyl)tetrahydro-3aH-cyclopenta[d] [1 ,3]dioxol-5-amine)), NC98-5 (4,7,13-tris(3-oxo-3-(undecylamino)propyl)-N1,N16-diundecyl-4,7,10,13-tetraazahexadecane-1,16-diamide), DODAP (1,2-dioleyl-3-dimethylammonium propane), HGT4003 (WO 2012/170889), ICE (WO 2011/068810), HGT5000 (U.S. Provisional Patent Application No. 61/617,468) or HGT5001 (cis or trans) (Provisional Patent Application No. 61/617,468), aminoalcohol lipidoids such as those disclosed in WO2010/053572, DOTAP (1,2-dioleyl-3-trimethylammonium propane), DOTMA (1,2-di-O-octadecenyl-3-trimethylammonium propane), DLinDMA (Heyes, J.; Palmer, L.; Bremner, K.; MacLachlan, I. "Cationic lipid saturation influences intracellular delivery of encapsulated nucleic acids" J. Contr. Rel. 2005, 107, 276-287), DLin-KC2-DMA (Semple, S.C. et al. "Rational Design of Cationic Lipids for siRNA Delivery" Nature Biotech. 2010, 28, 172-176), C12-200 (Love, K.T. et al. "Lipid-like materials for low-dose in vivo gene silencing" PNAS 2010, 107, 1864-1869).

In some embodiments, the percentage of cationic lipid in a liposome may be greater than 10%, greater than 20%, greater than 30%, greater than 40%, greater than 50%, greater than 60%, or greater than 70%. In some embodiments, cationic lipid(s) constitute(s) about 30-50 % (e.g., about 30-45%, about 30-40%, about 35-50%, about 35-45%, or about 35-40%) of the liposome by weight. In some embodiments, the cationic lipid (e.g., cKK-E12) constitutes about 30%, about 35%, about 40 %, about 45%, or about 50% of the liposome by molar ratio.

### Non-cationic/Helper Lipids

In some embodiments, provided liposomes contain one or more non-cationic ("helper") lipids. As used herein, the phrase "non-cationic lipid" refers to any neutral, zwitterionic or anionic lipid. As used herein, the phrase "anionic lipid" refers to any of a number of lipid species that carry a net negative charge at a selected H, such as physiological pH. Non-cationic lipids include, but are not limited to, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoyl-phosphatidylethanolamine (POPE), dioleoyl-phosphatidylethanolamine 4-(N-maleimidomethyl)-cyclohexane-1-carboxylate (DOPE-mal), dipalmitoyl phosphatidyl ethanolamine (DPPE), dimyristoylphosphoethanolamine (DMPE), distearoyl-phosphatidylethanolamine (DSPE), 16-O-monomethyl PE, 16-O-dimethyl PE, 18-1-trans PE, 1-stearoyl-2-oleoyl-phosphatidyethanolamine (SOPE), or a mixture thereof.

In some embodiments, such non-cationic lipids may be used alone, but are preferably used in combination with other excipients, for example, cationic lipids. In some embodiments, the non-cationic lipid may comprise a molar ratio of about 5% to about 90%, or about 10 % to about 70% of the total lipid present in a liposome. In some embodiments, a non-cationic lipid is a neutral lipid, i.e., a lipid that does not carry a net charge in the conditions under which the composition is formulated and/or administered. In some embodiments, the percentage of non-cationic lipid in a liposome may be greater than 5%, greater than 10%, greater than 20%, greater than 30%, or greater than 40%.

### Cholesterol-based Lipids

In some embodiments, provided liposomes comprise one or more cholesterol-based lipids. For example, suitable cholesterol-based cationic lipids include, for example, DC-Choi (N,N-dimethyl-N-ethylcarboxamidocholesterol), 1,4-bis(3-N-oleylamino-propyl)piperazine (Gao, et al. Biochem. Biophys. Res. Comm. 179, 280 (1991); Wolf et al. BioTechniques 23, 139 (1997); U.S. Pat. No. 5,744,335), or ICE. In some embodiments, the cholesterol-based lipid may comprise a molar ration of about 2% to about 30%, or about 5% to about 20% of the total lipid present in a liposome. In some embodiments, The percentage of cholesterol-based lipid in the lipid nanoparticle may be greater than 5, %, 10%, greater than 20%, greater than 30%, or greater than 40%.

### PEGylated Lipids

In some embodiments, provided liposomes comprise one or more PEGylated lipids. For example, the use of polyethylene glycol (PEG)-modified phospholipids and derivatized lipids such as derivatized ceramides (PEG-CER), including N-Octanoyl-Sphingosine-1-[Succinyl(Methoxy Polyethylene Glycol)-2000] (C8 PEG-2000 ceramide) is also contemplated by the present invention in combination with one or more of the cationic and other lipids together which comprise the liposome. Contemplated PEG-modified lipids include, but are not limited to, a polyethylene glycol chain of up to 5 kDa in length covalently attached to a lipid with alkyl chain(s) of C₆-C₂₀ length. In some embodiments, a PEG-modified or PEGylated lipid is PEGylated cholesterol or PEG-2K. The addition of such components may prevent complex aggregation and may also provide a means for increasing circulation lifetime and increasing the delivery of the lipid-nucleic acid composition to the target cell, (Klibanov et al. (1990) FEBS Letters, 268 (1): 235-237), or they may be selected to rapidly exchange out of the formulation *in vivo* (see U.S. Pat. No. 5,885,613).

In some embodiments, particularly useful exchangeable lipids are PEG-ceramides having shorter acyl chains (e.g., C₁₄ or C₁₈). The PEG-modified phospholipid and derivatized lipids of the present invention may comprise a molar ratio from about 0% to about 15%, about 0.5% to about 15%, about 1% to about 15%, about 4% to about 10%, or about 2% of the total lipid present in the liposome.

According to various embodiments, the selection of cationic lipids, non-cationic lipids and PEG-modified lipids which comprise the liposome, as well as the relative molar ratio of such lipids to each other, is based upon the characteristics of the selected lipid(s), the nature of the intended target cells and the characteristics of the mRNA to be delivered. Additional considerations include, for example, the saturation of the alkyl chain, as well as the size, charge, pH, pKa, fusogenicity and toxicity of the selected lipid(s). Thus the molar ratios may be adjusted accordingly.

In some embodiments, the cationic lipids, non-cationic lipids, cholesterol, and PEG-modified lipids can be combined at various relative molar ratios. For example, the ratio of cationic lipid (e.g., cKK-E12, C12-200, etc.) to non-cationic lipid (e.g., DOPE, etc.) to cholesterol-based lipid (e.g., cholesterol) to PEGylated lipid (e.g., DMG-PEG2K) may be between about 30-60:20-35:20-30:1-15, respectively. In some embodiments, the liposome comprises cKK-E12, DOPE, cholesterol, and DMG-PEG2K. In some embodiments, the liposome comprises cKK-E12, DOPE, cholesterol, and DMG-PEG2K at a ratio of 40:30:25:5, 50:25:20:5, 50:27:20:3, 40:30:20:10, 40:32:20:8, 40:32:25:3 or 40:33:25:2. In some embodiments, the liposome comprises cKK-E12, DSPC, cholesterol, and DMG-PEG2K. In some embodiments, the liposome comprises cKK-E12, DSPC, cholesterol, and DMG-PEG2K at a ratio of 40:30:25:5, 50:25:20:5, 40:30:20:10, or 40:32:20:8. In some embodiments, the liposome comprises C12-200, DOPE, cholesterol, and DMG-PEG2K. In some embodiments, the lipsome comprises C12-200, DOPE, cholesterol, and DMG-PEG2K at a ratio of 50:25:20:5, 50:20:25:5, 50:27:20:3 40:30:20:10, 40:30:25:5 or 40:32:20:8, 40:32:25:3 or 40:33:25:2.

### Formation of Liposomes

The liposomal transfer vehicles for use in the present invention can be prepared by various techniques which are presently known in the art. The liposomes for use in provided compositions can be prepared by various techniques which are presently known in the art. For example, multilamellar vesicles (MLV) may be prepared according to conventional techniques, such as by depositing a selected lipid on the inside wall of a suitable container or vessel by dissolving the lipid in an appropriate solvent, and then evaporating the solvent to leave a thin film on the inside of the vessel or by spray drying. An aqueous phase may then added to the vessel with a vortexing motion which results in the formation of MLVs. Uni-lamellar vesicles (ULV) can then be formed by homogenization, sonication or extrusion of the multi-lamellar vesicles. In addition, unilamellar vesicles can be formed by detergent removal techniques.

In certain embodiments, provided compositions comprise a liposome wherein the mRNA is associated on both the surface of the liposome and encapsulated within the same liposome. For example, during preparation of the compositions of the present invention, cationic liposomes may associate with the mRNA through electrostatic interactions.

In some embodiments, the one or more mRNA species may be encapsulated in the same liposome. In some embodiments, the one or more mRNA species may be encapsulated in different liposomes. In some embodiments, the mRNA is encapsulated in one or more liposomes, which differ in their lipid composition, molar ratio of lipid components, size, charge (Zeta potential), targeting ligands and/or combinations thereof. In some embodiments, the one or more liposome may have a different composition of cationic lipids, neutral lipid, PEG-modified lipid and/or combinations thereof. In some embodiments the one or more lipisomes may have a different molar ratio of cationic lipid, neutral lipid, cholesterol and PEG-modified lipid used to create the liposome.

The process of incorporation of a desired mRNA into a liposome is often referred to as "loading". Exemplary methods are described in Lasic, et al., FEBS Lett., 312: 255-258, 1992. The liposome-incorporated nucleic acids may be completely or partially located in the interior space of the liposome, within the bilayer membrane of the liposome, or associated with the exterior surface of the liposome membrane. The incorporation of a nucleic acid into liposomes is also referred to herein as "encapsulation" wherein the nucleic acid is entirely contained within the interior space of the liposome. The purpose of incorporating a mRNA into a transfer vehicle, such as a liposome, is often to protect the nucleic acid from an environment which may contain enzymes or chemicals that degrade nucleic acids and/or systems or receptors that cause the rapid excretion of the nucleic acids. Accordingly, in some embodiments, a suitable delivery vehicle is capable of enhancing the stability of the mRNA contained therein and/or facilitate the delivery of mRNA to the target cell or tissue.

### Liposome Size

Suitable liposomes in accordance with the present invention may be made in various sizes. In some embodiments, a suitable nanoparticle has a size of or less than about 100 nm (e.g., of or less than about 90 nm, 80 nm, 70 nm, 60 nm, 50 nm, 40 nm, 30 nm, or 20 nm). In some embodiments, the liposome has a size of or less than about 60 nm (e.g., of or less than about 55 nm, of or less than about 50 nm, of or less than about 45 nm, of or less than about 40 nm, of or less than about 35 nm, of or less than about 30 nm, or of or less than about 25 nm). In some embodiments, a suitable liposome has a size ranging from about 10 - 100 nm (e.g., ranging from about 10 - 90 nm, 10 - 80 nm, 10 - 70 nm, 10 - 60 nm, 10 - 50 nm, 10-40 nm, or 10-30 nm). Liposomes with a size of 60 - 100 nm (Z_{average}) and in particular nanoparticles with a size of 70 - 90 nm (Z_{average}) may be used in practicing the invention. The polydispersity index (PDI) of the liposomes is typically in the range of 0.1 to 0.5. In a particular embodiment, a PDI is below 0.2. Typically, the PDI is determined by dynamic light scattering.

A variety of alternative methods known in the art are available for sizing of a population of liposomes. One such sizing method is described in U.S. Pat. No. 4,737,323. Sonicating a liposome suspension either by bath or probe sonication produces a progressive size reduction down to small ULV less than about 0.05 microns in diameter. Homogenization is another method that relies on shearing energy to fragment large liposomes into smaller ones. In a typical homogenization procedure, MLV are recirculated through a standard emulsion homogenizer until selected liposome sizes, typically between about 0.1 and 0.5 microns, are observed. The size of the liposomes may be determined by quasi-electric light scattering (QELS) as described in Bloomfield, Ann. Rev. Biophys. Bioeng., 10:421-150 (1981). Average liposome diameter may be reduced by sonication of formed liposomes. Intermittent sonication cycles may be alternated with QELS assessment to guide efficient liposome synthesis.

### Pharmaceutical Compositions and Administration

To facilitate expression of mRNA *in vivo,* delivery vehicles such as liposomes can be formulated in combination with one or more additional nucleic acids, carriers, targeting ligands or stabilizing reagents, or in pharmacological compositions where it is mixed with suitable excipients. Techniques for formulation and administration of drugs may be found in "Remington's Pharmaceutical Sciences," Mack Publishing Co., Easton, Pa., latest edition.

mRNA-containing compositions may be administered and dosed in accordance with current medical practice, taking into account the clinical condition of the subject, the site and method of administration, the scheduling of administration, the subject's age, sex, body weight and other factors relevant to clinicians of ordinary skill in the art. Intravitreal administration is used to deliver the mRNA to the eye. Intravitreal administration is used where it is desired that expression of the mRNA is restricted to the eye, e.g. where the mRNA encodes a protein that is expressed solely in the eye or where the mRNA encodes a therapeutic protein whose activity is particularly restricted to the eye (e.g., where the protein encodes a VEGF antagonist such as an anti-VEGF antibody or a soluble VEGF receptor). The "effective amount" for the purposes herein may be determined by such relevant considerations as are known to those of ordinary skill in experimental clinical research, pharmacological, clinical and medical arts. In some embodiments, the amount administered is effective to achieve at least some stabilization, improvement or elimination of symptoms and other indicators as are selected as appropriate measures of disease progress, regression or improvement by those of skill in the art. In some embodiments, a suitable amount and dosing regimen is one that results in protein (e.g., antibody) expression or activity in the eye. In some embodiments, the expression and/or activity of the protein is detected in corneal cells, scleral cells, choroid plexus epithelial cells, ciliary body cells, retinal cells, and/or vitreous humour. In some embodiments, the expression and/or activity of the protein is detected in the posterior region of the eye. In some embodiments, the expression and/or activity of the protein is detected in the anterior region of the eye. In some embodiments, the expression and/or activity of the protein is detected in both the posterior and anterior regions of the eye. In some embodiments, the expression and/or activity of the protein is detected by blood sampling. In some embodiments, the expression and/or activity of the protein is detected by sampling a vitreous humor. In some embodiments, the expression and/or activity of the protein is detectable about 6 hours, 12 hours, 18 hours, 24 hours, 2 days, 3 days, 4 days, 5 days, 6 days, 1 week, 2 weeks, 3 weeks, 4 weeks, 1 month, 2 months, 3 months, 4 months, 5 months, 6 months or longer after a single administration.

Provided methods contemplate single as well as multiple administrations of a therapeutically effective amount of mRNA or a composition described herein. mRNA or a composition described herein can be administered at regular intervals, depending on the nature, severity and extent of the subject's condition. In some embodiments, a therapeutically effective amount of mRNA or a composition described herein may be administered periodically at regular intervals (e.g., once every year, once every six months, once every five months, once every four months, once every three months, bimonthly (once every two months), monthly (once every month), once every three weeks, biweekly (once every two weeks), weekly, once every three days, once every two days, daily or continuously). Typical intervals include once every month and every two months. In some embodiments, mRNA or a composition described herein may be administered at variable intervals.

### EXAMPLES

While certain compounds, compositions and methods have been described with specificity in accordance with certain embodiments, the following examples serve only to illustrate the invention and are not intended to limit the same.

### Example 1. Exemplary Liposome Formulations for mRNA Delivery and Expression

This example provides exemplary liposome formulations for effective delivery and expression of mRNA *in vivo.*

### Lipid Materials

The formulations described herein consisted of a multi-component lipid mixture of varying ratios employing one or more cationic lipids, helper lipids and PEGylated lipids designed to encapsulate various nucleic acid-based materials. Cationic lipids can include (but not exclusively) DOTAP (1,2-dioleyl-3-trimethylammonium propane), DODAP (1,2-dioleyl-3-dimethylammonium propane), DOTMA (1,2-di-O-octadecenyl-3-trimethylammonium propane), DLinDMA (Heyes, J.; Palmer, L.; Bremner, K.; MacLachlan, I. "Cationic lipid saturation influences intracellular delivery of encapsulated nucleic acids" J. Contr. Rel. 2005, 107, 276-287), DLin-KC2-DMA (Semple, S.C. et al. "Rational Design of Cationic Lipids for siRNA Delivery" Nature Biotech. 2010, 28, 172-176), C12-200 (Love, K.T. et al. "Lipid-like materials for low-dose in vivo gene silencing" PNAS 2010, 107, 1864-1869), MD1 (3,6-bis(4-(bis(2-hydroxydodecyl)amino)butyl)piperazine-2,5-dione), cKK-E12, HGT5000, HGT5001, HGT4003, ICE, dialkylamino-based, imidazole-based, guanidinium-based, etc. Helper lipids can include (but not exclusively) DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), DOPG (,2-dioleoyl-sn-glycero-3-phospho-(1'-rac-glycerol)), cholesterol, etc. The PEGylated lipids can include (but not exclusively) a poly(ethylene) glycol chain of up to 5 kDa in length covalently attached to a lipid with alkyl chain(s) of C6-C20 length. Polyethyleneimine can be linear or branched. For branched PEI, 25 kDa is preferred but not exclusive.

### Messenger RNA Material

Codon-optimized human firefly luciferase (FFL) or human argininosuccinate synthetase (ASS1) messenger RNA was synthesized by *in vitro* transcription from a plasmid DNA template encoding the gene, which was followed by the addition of a 5' cap structure (Cap 1) (Fechter, P.; Brownlee, G.G. "Recognition of mRNA cap structures by viral and cellular proteins" J. Gen. Virology 2005, 86, 1239-1249) and a 3' poly(A) tail of approximately 250 nucleotides (SEQ ID NO: 8) in length as determined by gel electrophoresis. 5' and 3' untranslated regions present in each mRNA product are represented as X and Y, respectively, and defined as stated (*vide infra*).
*Codon-Optimized Firefly Luciferase (FFL) mRNA:*
*Codon-Optimized Human Argininosuccinate Synthetase (ASS1) mRNA:*
*5' and 3' UTR Sequences*
   X (5' UTR Sequence)
Y (3' UTR Sequence) OR

### Exemplary Formulation Protocols

### A. cKK-E12

Aliquots of 50 mg/mL ethanolic solutions of cKK-E12, DOPE, cholesterol and DMG-PEG2K were mixed and diluted with ethanol to 3 mL final volume. Separately, an aqueous buffered solution (10 mM citrate/150 mM NaCl, pH 4.5) of FFL or ASS1 mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C. Final concentration 1.0 mg/mL FFL mRNA (encapsulated). Zₐᵥₑ 80 nm; PDI 0.12. Final concentration 2.0 mg/mL ASS1 mRNA (encapsulated). Zₐᵥₑ 82 nm; PDI: 0.14.

### B. C12-200

Aliquots of 50 mg/mL ethanolic solutions of C12-200, DOPE, cholesterol and DMG-PEG2K were mixed and diluted with ethanol to 3 mL final volume. Separately, an aqueous buffered solution (10 mM citrate/150 mM NaCl, pH 4.5) of FFL mRNA was prepared from a 1 mg/mL stock. The lipid solution was injected rapidly into the aqueous mRNA solution and shaken to yield a final suspension in 20% ethanol. The resulting nanoparticle suspension was filtered, diafiltrated with 1x PBS (pH 7.4), concentrated and stored at 2-8°C. Final concentration 1.0 mg/mL FFL mRNA (encapsulated). Zₐᵥₑ 77 nm; PDI: 0.14.

### C. PEI

25 kDa branched PEI solution at a concentration of 1.34 mg/mL (pH 5.0) was mixed with equal volumes of FFL mRNA (1.0 mg/mL). The resultant formulation was stored at 2-8°C. Final concentration 0.50 mg/mL FFL mRNA.

### Example 2. Analysis of protein produced via intravitreal delivery of mRNA-loaded nanoparticles

This example illustrates exemplary methods of administering FFL or ASS1 mRNA-loaded liposome nanoparticles and methods for analyzing delivered mRNA and subsequently expressed FFL or ASS1 protein in various target tissues *in vivo.*

All studies were performed using either male CD-1 mice or Sprague-Dawley rats. Samples were introduced to mice by a single bolus tail-vein injection of encapsulated FFL or ASS1 mRNA. Samples were introduced to rats by direct intravitreal injection of encapsulated ASS1 mRNA-loaded lipid nanoparticles.

### Injection protocol

Animals were anesthetized with intraperitoneal injection of ketamine 60 - 150 mg/kg and xylazine 6 - 32 mg/kg mixture. Test materials were injected via intravitreal injection into the left eye only. Yohimbine (2 mg/mL, 0.01 - 0.02 ml/animal, IP) was administered to the animals following dose administration to enhance recovery from anesthesia.

### Live imaging of animals

At 6 and 24 hours post completion of dose administration (± 5%), all animals were subjected to an imaging session with IVIS Lumina equipment. The animals were treated with ketamine/xylazine [40-100mg/kg/5-15mg/kg (rats), 60-150mg/kg/6-32mg/kg (mice)] via IP injection. Following this, animals were injected with up to 5 µL/animal (mice) or up to 10 µL/animal (rats) of luciferin in PBS at 60 mg/mL via intravitreous injection. The luciferin was allowed to distribute for 5-10 minutes.

### Isolation of organ tissues for analysis

Following the imaging session, all animals were euthanized by CO₂ asphyxiation, followed by thoracotomy. For half of the animals/group, the left eyeball was harvested. Half of the eyeballs were stored as one histology cassette per animal. All histology cassettes were stored at ambient conditions in 10% NBF for at least 24 hours, but no more than 72 hours, before being transferred into 70% alcohol solution and processed for histology (paraffin). The other eyeballs/group were placed into individual tubes, then snap frozen in liquid nitrogen and stored at approximately -70°C.

For the remaining half of the animals from each group, the retinas of the left eye were harvested. Half of the retinas/group were stored at ambient conditions in 10% NBF for at least 24 hours, but no more than 72 hours, before being transferred into 70% alcohol solution. Fixed retinas were transferred to PBS and mounted on one (1) microscope slide per group and stored at 5 ± 3°C. The other retinas/group were placed into individual tubes, then snap frozen in liquid nitrogen and stored at approximately -70°C.

### Enzyme-Linked Immunosorbent Assay (ELISA) Analysis

Standard ELISA procedures were followed employing mouse anti-ASS1 2D1-2E12 IgG as the capture antibody with rabbit anti-ASS1 #3285 IgG as the secondary (detection) antibody (Shire Human Genetic Therapies). Horseradish peroxidase (HRP)-conjugated goat anti-rabbit IgG was used for activation of the 3,3',5,5'-tetramethylbenzidine (TMB) substrate solution. The reaction was quenched using 2N H2SO4 after 20 minutes. Detection was monitored via absorption (450 nm) on a Molecular Device SpectraMax instrument. Untreated organs and human ASS1 protein were used as negative and positive controls, respectively.

### Example 3. Efficient in vivo protein production in the eye

This example demonstrates that administration of mRNA encoding FFL or ASS1 results in successful *in vivo* protein production in the eye. ASS1 was chosen partly because ASS1 is a protein expressed in the eye under physiological conditions. Thus, this example establishes that mRNA therapy may be used to deliver physiologically relevant protein to replace, increase or supplement endogenous protein activity in the eye.

### In vivo firefly luciferase protein production results

The production of firefly luciferase protein via codon-optimized FFL mRNA-loaded lipid and polymeric nanoparticles was tested in CD-1 mice as a single, bolus intravitreal injection. Figures 1-4 represent the luminescence detected via *in vivo* bioluminescent imaging using an IVIS imager. Such luminescence was measured 6 and 24 hours post injection of FFL mRNA nanoparticles into wild type mice. Table 1 represents luminescence values after luciferin administration (total flux (photon/sec), 5 minutes post-luciferin). The mice were sacrificed twenty-four hours post-injection and organs were harvested (as described above).

**Table 1. Quantitation of total luminescent flux (photons/sec) produced from active firefly luciferase protein derived from intravitreal administration of FFL mRNA-loaded nanoparticles. Values are reflective of protein produced 24 hours post-administration of test article. Total flux is measured five minutes after luciferin substrate administration.**

| **Carrier (Cationic Component)** | **mRNA** | **Dose (µg)** | **Total Flux (photon/sec)** |
|---|---|---|---|
| cKK-E12 | FFL | 5.0 | 70,000,000 |
| C12-200 | FFL | 5.0 | 59,100,000 |
| PEI (25 kDA branched) | FFL | 2.5 | 2,350,000 |
| Saline | N/A | N/A | 22,800 |

### In vivo human ASS1 protein production results

The production of human ASS1 protein via codon-optimized hASS1 mRNA-loaded lipid nanoparticles was tested in CD-1 mice and Sprague-Dawley rats as a single, bolus intravitreal injection. Figure 5 represents the amount of human ASS1 protein detected in the eye via ELISA when treating mice with either human ASS1 mRNA-loaded MD1-based lipid nanoparticles (5 micrograms, based on encapsulated mRNA) or control formulations (STOP mRNA and/or saline). The mice were sacrificed twenty-four hours post-injection and organs were harvested (as described above).

A clear signal was detected when measuring eye levels of human ASS1 protein via ELISA. This is in contrast to complete lack of signal when analyzing eyeballs treated with either STOP mRNA control or saline (Figure 5). These data demonstrate the ability of the lipid nanoparticles to accumulate in eye cells and release the mRNA payload to process this exogenous mRNA via translation to produce human ASS1 protein.

Such protein production was also achieved in a second rodent species, Sprague-Dawley rats. As depicted in Figure 6, treatment of rats via direct intravitreal injection with ASS1 mRNA-loaded lipid nanoparticles (20 micrograms, based on encapsulated mRNA) resulted in detectable levels of human ASS1 protein within the treated eye.

### EQUIVALENTS

Those skilled in the art will recognize, or be able to ascertain using no more than routine experimentation, many equivalents to the specific embodiments described herein. The scope of the present invention is not intended to be limited to the above description, but rather is as set forth in the claims.

## Claims

1. A composition comprising an mRNA encoding a therapeutic protein for use in treating an eye disease, disorder or condition in a subject in need thereof, wherein the composition is administered into an eye of the subject via intravitreal administration such that the administration of the composition results in expression and/or activity of the therapeutic protein encoded by the mRNA in the eye, wherein the mRNA is codon-optimized, has a length of 0.5 kb to 5 kb and is encapsulated within a liposome, wherein the liposome comprises one or more cationic lipids, one or more non-cationic lipids, one or more cholesterol-based lipids and one or more PEG-modified lipids.

2. The composition for use according to claim 1, wherein the expression and/or activity of the therapeutic protein is detected in corneal cells, scleral cells, choroid plexus epithelial cells, ciliary body cells, retinal cells, and/or vitreous humour, optionally wherein the expression and/or activity of the therapeutic protein is detected by (a) blood sampling, or (b) sampling a vitreous humor.

3. The composition for use according to claim 1 or 2, wherein the expression and/or activity of the therapeutic protein is detectable about 6 hours, 12 hours, 18 hours, 24 hours, 2 days, 3 days, 4 days, 5 days, 6 days, 1 week, 2 weeks, 3 weeks, 4 weeks, or 1 month post-administration.

4. The composition for use according to any one of the preceding claims, wherein the mRNA has a length of 1 kb to 3 kb.

5. The composition for use according to any one of the preceding claims, wherein the therapeutic protein encoded by the mRNA (a) normally functions in the eye, (b) is an antibody, or (c) is a soluble receptor, optionally wherein the antibody encoded by the mRNA is an anti-VEGF antibody, anti-TNFa antibody, anti-IL-6 antibody, anti-ICAM-1 antibody, anti-VCAM-1, or soluble VEGF receptor.

6. The composition for use according to any one of the preceding claims, wherein the eye disease, disorder or condition is selected from AMD, PU, BRVO, CRVO, DME, CME, UME, CMV retinitis, endophthalmitis, inflammation, glaucoma, macular degeneration, scleritis, choriotetinitis, Dry eye syndrome, Stargardt disease, Norris disease, Coat's disease, persistent hyperplastic primary vitreous, familial exudative vitreoretinopathy, Leber congenital amaurosis, Retinitis Pigmentosis, X-linked retinoschesis, Leber's hereditary optic neurophathy (LHON), and uveitis, optionally wherein the composition is administered (a) once a week, (b) twice a month, or (c) once a month.

7. The composition for use according to any one of the preceding claims, wherein the one or more cationic lipids are selected from the group consisting of C12-200, MC3, DLinDMA, DLinkC2DMA, cKK-E12, ICE (Imidazole-based), HGT5000, HGT5001, DODAC, DDAB, DMRIE, DOSPA, DOGS, DODAP, DODMA and DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, HGT4003, and combinations thereof, optionally wherein the one or more cationic lipids comprise cKK-E12:

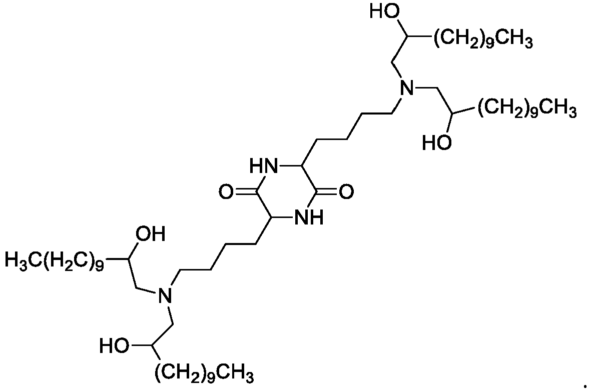

8. The composition for use according to any one of the preceding claims, wherein the one or more non-cationic lipids are selected from the group consisting of DSPC (1,2-distearoyl-sn-glycero-3-phosphocholine), DPPC (1,2-dipalmitoyl-sn-glycero-3-phosphocholine), DOPE (1,2-dioleyl-sn-glycero-3-phosphoethanolamine), DOPC (1,2-dioleyl-sn-glycero-3-phosphotidylcholine) DPPE (1,2-dipalmitoyl-sn-glycero-3-phosphoethanolamine), DMPE (1,2-dimyristoyl-sn-glycero-3-phosphoethanolamine), DOPG (,2-dioleoyl-*sn*-glycero-3-phospho-(1'-*rac*-glycerol)) and combinations thereof.

9. The composition for use according to any one of the preceding claims, wherein the one or more cholesterol-based lipids is cholesterol or PEGylated cholesterol.

10. The composition for use according to any one of the preceding claims, wherein the one or more PEG-modified lipids comprise a poly(ethylene) glycol chain of up to 5 kDa in length covalently attached to a lipid with alkyl chain(s) of C6-C20 length.

11. The composition for use according to any one of the preceding claims, wherein the cationic lipid constitutes about 30-70 % of the liposome by molar ratio.

12. The composition for use according to any one of the preceding claims , wherein the liposome comprises:
(a) cKK-E12, DOPE, cholesterol, and DMG-PEG2K, optionally at a ratio of 40:30:25:5, 50:25:20:5, 50:27:20:3, 40:30:20:10, 40:32:20:8, 40:32:25:3 or 40:33:25:2;
(b) cKK-E12, DSPC, cholesterol, and DMG-PEG2K, optionally at a ratio of 40:30:25:5, 50:25:20:5, 50:27:20:3, 40:30:20:10, 40:32:20:8, 40:32:25:3 or 40:33:25:2; or
(c) C12-200, DOPE, cholesterol, and DMG-PEG2K, optionally at a ratio of 50:25:20:5, 50:20:25:5, 50:27:20:3, 40:30:20:10, 40:30:25:5, 40:32:20:8, 40:32:25:3 or 40:33:25:2.

13. The composition for use according to any one of the preceding claims, wherein the liposome has a size less than about 60 nm.

14. The composition for use according to any one of the preceding claims, wherein the mRNA comprises one or more modified nucleotides, optionally wherein the one or more modified nucleotides comprise pseudouridine, N-1-methyl-pseudouridine, 2-aminoadenosine, 2-thiothymidine, inosine, pyrrolo-pyrimidine, 3-methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine, O(6)-methylguanine, and/or 2-thiocytidine.

15. The composition for use according to any one of claims 1-13, wherein the mRNA is unmodified.

## Patentansprüche

1. Zusammensetzung, umfassend eine mRNA, die ein therapeutisches Protein codiert, zur Verwendung bei der Behandlung einer Augenkrankheit, -störung bzw. eines Augenleidens bei einem diese benötigenden Individuum, wobei die Zusammensetzung in ein Auge des Individuums über intravitreale Verabreichung verabreicht wird, so dass die Verabreichung der Zusammensetzung zu Expression und/oder Aktivität des durch die mRNA codierten therapeutischen Proteins im Auge führt, wobei die mRNA codonoptimiert ist, eine Länge von 0,5 kb bis 5 kb aufweist und in einem Liposom verkapselt ist, wobei das Liposom ein oder mehrere kationische Lipide, ein oder mehrere nichtkationische Lipide, ein oder mehrere Lipide auf Cholesterinbasis und ein oder mehrere PEG-modifizierte Lipide umfasst.

2. Zusammensetzung zur Verwendung nach Anspruch 1, wobei die Expression und/oder Aktivität des therapeutischen Proteins in Hornhautzellen, Lederhautzellen, Epithelzellen des Plexus choroideus, Ziliarkörperzellen, Netzhautzellen und/oder im Glaskörper nachgewiesen wird, gegebenenfalls wobei die Expression und/oder Aktivität des therapeutischen Proteins durch (a) Blutprobennahme oder (b) Entnahme einer Glaskörperprobe nachgewiesen wird.

3. Zusammensetzung zur Verwendung nach Anspruch 1 oder 2, wobei die Expression und/oder Aktivität des therapeutischen Proteins etwa 6 Stunden, 12 Stunden, 18 Stunden, 24 Stunden, 2 Tage, 3 Tage, 4 Tage, 5 Tage, 6 Tage, 1 Woche, 2 Wochen, 3 Wochen, 4 Wochen oder 1 Monat nach Verabreichung nachweisbar ist.

4. Zusammensetzung zur Verwendung nach einem der vorhergehenden Ansprüche, wobei die mRNA eine Länge von 1 kb bis 3 kb aufweist.

5. Zusammensetzung zur Verwendung nach einem der vorhergehenden Ansprüche, wobei das durch die mRNA codierte therapeutische Protein (a) normalerweise im Auge funktionsfähig ist, (b) ein Antikörper ist oder (c) ein löslicher Rezeptor ist, gegebenenfalls wobei es sich bei dem durch die mRNA codierten Antikörper um einen Anti-VEGF-Antikörper, Anti-TNFa-Antikörper, Anti-IL-6-Antikörper, Anti-ICAM-1-Antikörper, Anti-VCAM-1 oder löslichen VEGF-Rezeptor handelt.

6. Zusammensetzung zur Verwendung nach einem der vorhergehenden Ansprüche, wobei die Augenkrankheit, -störung bzw. das Augenleiden aus AMD, PU, BRVO, CRVO, DME, CME, UME, CMV-Retinitis, Endophthalmitis, Entzündung, Glaukom, Makuladegeneration, Skleritis, Choriotetinitis, Keratoconjunctivitis sicca, Morbus Stargardt, Norris-Syndrom, Morbus Coats, persistierender hyperplastischer primärer Vitreus, familiärer exsudativer Vitreoretinopathie, Leberscher kongenitaler Amaurose, Retinitis pigmentosis, X-verknüpfter Retinoschisis, Leberscher Optikusatropie (LHON) und Uveitis ausgewählt ist, gegebenenfalls wobei die Zusammensetzung (a) einmal pro Woche, (b) zweimal pro Monat oder (c) einmal pro Monat verabreicht wird.

7. Zusammensetzung zur Verwendung nach einem der vorhergehenden Ansprüche, wobei das eine bzw. die mehreren kationischen Lipide aus der Gruppe bestehend aus C12-200, MC3, DLinDMA, DLinkC2DMA, cKK-E12, ICE (auf Imidazolbasis), HGT5000, HGT5001, DODAC, DDAB, DMRIE, DOSPA, DOGS, DODAP, DODMA und DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, HGT4003 und Kombinationen davon ausgewählt sind, gegebenenfalls wobei das eine bzw. die mehreren kationischen Lipide cKK-E12 umfassen:

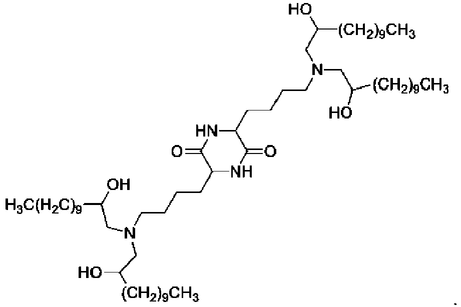

8. Zusammensetzung zur Verwendung nach einem der vorhergehenden Ansprüche, wobei das eine bzw. die mehreren nichtkationischen Lipide aus der Gruppe bestehend aus DSPC (1,2-Distearoyl-sn-glycero-3-phosphocholin), DPPC (1,2-Dipalmitoyl-sn-glycero-3-phosphocholin), DOPE (1,2-Dioleyl-sn-glycero-3-phosphoethanolamin), DOPC (1,2-Dioleyl-sn-glycero-3-phosphotidylcholin) DPPE (1,2-Dipalmitoyl-sn-gly-cero-3-phosphoethanolamin), DMPE (1,2-Dimyristoyl-sn-glycero-3-phosphoethanolamin), DOPG (,2-Di-oleoyl-sn-glycero-3-phospho-(1'-rac-glycerol)) und Kombinationen davon ausgewählt sind.

9. Zusammensetzung zur Verwendung nach einem der vorhergehenden Ansprüche, wobei es sich bei dem einen bzw. den mehreren Lipiden auf Cholesterinbasis um Cholesterin oder PEGyliertes Cholesterin handelt.

10. Zusammensetzung zur Verwendung nach einem der vorhergehenden Ansprüche, wobei das eine bzw. die mehreren PEG-modifizierten Lipide eine Polyethylenglykol-Kette mit einer Länge von bis zu 5 kDa, die an ein Lipid mit einer Alkylkette bzw. Alkylketten mit C6-C20-Länge kovalent gebunden ist, umfassen.

11. Zusammensetzung zur Verwendung nach einem der vorhergehenden Ansprüche, wobei das kationische Lipid nach Molverhältnis etwa 30-70 % des Liposoms ausmacht.

12. Zusammensetzung zur Verwendung nach einem der vorhergehenden Ansprüche, wobei das Liposom Folgendes umfasst:
(a) cKK-E12, DOPE, Cholesterin und DMG-PEG2K, gegebenenfalls in einem Verhältnis von 40:30:25:5, 50:25:20:5, 50:27:20:3, 40:30:20:10, 40:32:20:8, 40:32:25:3 oder 40:33:25:2;
(b) cKK-E12, DSPC, Cholesterin und DMG-PEG2K, gegebenenfalls in einem Verhältnis von 40:30:25:5, 50:25:20:5, 50:27:20:3, 40:30:20:10, 40:32:20:8, 40:32:25:3 oder 40:33:25:2; oder
(c) C12-200, DOPE, Cholesterin und DMG-PEG2K, gegebenenfalls in einem Verhältnis von 50:25:20:5, 50:20:25:5, 50:27:20:3, 40:30:20:10, 40:30:25:5, 40:32:20:8, 40:32:25:3 oder 40:33:25:2.

13. Zusammensetzung zur Verwendung nach einem der vorhergehenden Ansprüche, wobei das Liposom eine Größe von weniger als etwa 60 nm aufweist.

14. Zusammensetzung zur Verwendung nach einem der vorhergehenden Ansprüche, wobei die mRNA ein oder mehrere modifizierte Nukleotide umfasst, gegebenenfalls wobei das eine bzw. die mehreren modifizierten Nukleotide Pseudouridin, N-1-Methylpseudouridin, 2-Aminoadenosin, 2-Thiothymidin, Inosin, Pyrrolopyrimidin, 3-Methyladenosin, 5-Methylcytidin, C-5-Propinylcytidin, C-5-Propinyluridin, 2-Aminoadenosin, C5-Bromuridin, C5-Fluoruridin, C5-Ioduridin, C5-Propinyluridin, C5-Propinylcytidin, C5-Methylcytidin, 2-Aminoadenosin, 7-Deazaadenosin, 7-Deazaguanosin, 8-Oxoadenosin, 8-Oxoguanosin, O(6)-Methylguanin und/oder 2-Thiocytidin umfassen.

15. Zusammensetzung zur Verwendung nach einem der Ansprüche 1-13, wobei die mRNA unmodifiziert ist.

## Revendications

1. Composition comprenant un ARNm codant pour une protéine thérapeutique pour utilisation dans le traitement d'une maladie, un trouble ou une affection oculaire chez un sujet en ayant besoin, la composition étant administrée dans un oeil du sujet par administration intravitréenne de sorte que l'administration de la composition conduise à l'expression et/ou l'activité de la protéine thérapeutique codée par l'ARNm dans l'oeil, l'ARNm étant optimisé en codons, ayant une longueur de 0,5 kb à 5 kb et étant encapsulé dans un liposome, le liposome comprenant un ou plusieurs lipides cationiques, un ou plusieurs lipides non cationiques, un ou plusieurs lipides à base de cholestérol et un ou plusieurs lipides modifiés par PEG.

2. Composition pour utilisation selon la revendication 1, l'expression et/ou l'activité de la protéine thérapeutique étant détectée dans des cellules cornéennes, des cellules sclérales, des cellules épithéliales du plexus choroïde, des cellules de corps ciliaire, des cellules rétiniennes et/ou l'humeur vitrée, l'expression et/ou l'activité de la protéine thérapeutique étant facultativement détectées par (a) prélèvement sanguin, ou (b) prélèvement d'humeur vitrée.

3. Composition pour utilisation selon la revendication 1 ou 2, l'expression et/ou l'activité de la protéine thérapeutique étant détectable environ 6 heures, 12 heures, 18 heures, 24 heures, 2 jours, 3 jours, 4 jours, 5 jours, 6 jours, 1 semaine, 2 semaines, 3 semaines, 4 semaines ou 1 mois après l'administration.

4. Composition pour utilisation selon l'une quelconque des revendications précédentes, dans laquelle l'ARNm a une longueur de 1 kb à 3 kb.

5. Composition pour utilisation selon l'une quelconque des revendications précédentes, la protéine thérapeutique codée par l'ARNm (a) fonctionnant normalement dans l'oeil, (b) étant un anticorps ou (c) étant un récepteur soluble, l'anticorps codé par l'ARNm étant facultativement un anticorps anti-VEGF, un anticorps anti-TNFα, un anticorps anti-IL-6, un anticorps anti-ICAM-1, anti-VCAM-1 ou un récepteur soluble de VEGF.

6. Composition pour utilisation selon l'une quelconque des revendications précédentes, la maladie, le trouble ou l'affection oculaire étant choisie parmi DMLA, PU, BRVO, OVCR, DME, CME, UME, CMV, la rétinite, l'endophtalmite, une inflammation, le glaucome, la dégénérescence maculaire, la sclérite, la choriorétinite, le syndrome de sécheresse oculaire, la maladie de Stargardt, la maladie de Norris, la maladie de Coat, le corps vitré primaire hyperplasique persistant, la vitréorétinopathie exsudative familiale, l'amaurose congénitale de Leber, la rétinite pigmentaire, le rétinoschisis lié à l'X, la neuropathie optique héréditaire de Leber (LHON) et l'uvéite, la composition étant administrée (a) une fois par semaine, (b) deux fois par mois, ou (c) une fois par mois.

7. Composition pour utilisation selon l'une quelconque des revendications précédentes, dans laquelle les un ou plusieurs lipides cationiques sont choisis dans le groupe constitué de C12-200, MC3, DLinDMA, DLinkC2DMA, cKK-E12, ICE (à base d'imidazole), HGT5000, HGT5001, DODAC, DDAB, DMRIE, DOSPA, DOGS, DODAP, DODMA et DMDMA, DODAC, DLenDMA, DMRIE, CLinDMA, CpLinDMA, DMOBA, DOcarbDAP, DLinDAP, DLincarbDAP, DLinCDAP, KLin-K-DMA, DLin-K-XTC2-DMA, HGT4003, et des combinaisons de ceux-ci, les un ou plusieurs lipides cationiques comprenant facultativement cKK-E12:

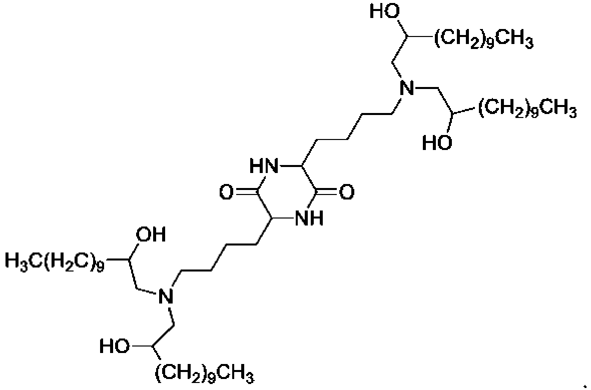

8. Composition pour utilisation selon l'une quelconque des revendications précédentes, dans laquelle les un ou plusieurs lipides cationiques sont choisis dans le groupe constitué de DSPC (1,2-distéaroyl-sn-glycéro-3-phosphocholine), DPPC (1,2-dipalmitoyl-sn-glycéro-3-phosphocholine), DOPE (1,2-dioléyl-sn-glycéro-3-phosphoéthanolamine), DOPC (1,2-dioléyl-sn-glycéro-3-phosphotidylcholine) DPPE (1,2-dipalmitoyl-sn-glycéro-3-phosphoéthanolamine), DMPE (1,2-dimyristoyl-sn-glycéro-3-phosphoéthanolamine), DOPG (,2-dioléoyl-*sn*-glycéro-3-phospho-(1'-*rac*-glycérol)) et des combinaisons de ceux-ci.

9. Composition pour utilisation selon l'une quelconque des revendications précédentes, dans laquelle les un ou plusieurs lipides à base de cholestérol sont le cholestérol ou le cholestérol PEGylé.

10. Composition pour utilisation selon l'une quelconque des revendications précédentes, dans laquelle les un ou plusieurs lipides modifiés par PEG comprennent une chaîne de poly(éthylène)glycol de jusqu'à 5 kDa de longueur liée de façon covalente à un lipide avec une ou plusieurs chaîne(s) alkyle de C6 à C20 de longueur.

11. Composition pour utilisation selon l'une quelconque des revendications précédentes, dans laquelle le lipide cationique constitue environ 30 à 70 % du liposome en rapport molaire.

12. Composition pour utilisation selon l'une quelconque des revendications précédentes, dans laquelle le liposome comprend :
(a) cKK-E12, DOPE, cholestérol et DMG-PEG2K, facultativement à un rapport de 40:30:25:5, 50:25:20:5, 50:27:20:3, 40:30:20:10, 40:32:20:8, 40:32:25:3 ou 40:33:25:2 ;
(b) cKK-E12, DSPC, cholestérol et DMG-PEG2K, facultativement à un rapport de 40:30:25:5, 50:25:20:5, 50:27:20:3, 40:30:20:10, 40:32:20:8, 40:32:25:3 ou 40:33:25:2 ; ou
(c) C12-200, DOPE, cholestérol et DMG-PEG2K, facultativement à un rapport de 50:25:20:5, 50:20:25:5, 50:27:20:3, 40:30:20:10, 40:30:25:5, 40:32:20:8, 40:32:25:3 ou 40:33:25:2.

13. Composition pour utilisation selon l'une quelconque des revendications précédentes, dans laquelle le liposome a une taille inférieure à environ 60 nm.

14. Composition pour utilisation selon l'une quelconque des revendications précédentes, dans laquelle l'ARNm comprend un ou plusieurs nucléotides modifiés, facultativement dans laquelle les un ou plusieurs nucléotides modifiés comprennent la pseudo-uridine, la N-1-méthyl-pseudo-uridine, la 2-aminoadénosine, la 2-thiothymidine, l'inosine, la pyrrolopyrimidine, la 3-méthyladénosine, la 5-méthylcytidine, la C5-propynyl-cytidine, la C5-propynyl-uridine, la 2-aminoadénosine, la C5-bromo-uridine, la C5-fluoro-uridine, la C5-iodo-uridine, la C5-propynyl-uridine, la C5-propynyl-cytidine, la C5-méthylcytidine, la 2-aminoadénosine, la 7-déaza-adénosine, la 7-déazaguanosine, la 8-oxoadénosine, la 8-oxoguanosine, la O(6)-méthylguanine et/ou la 2-thiocytidine.

15. Composition pour utilisation selon l'une quelconque des revendications 1 à 13, dans laquelle l'ARNm est non modifié.
